(19) Europäisches Patentamt / European Patent Office / Office européen des brevets

(11) **EP 4 010 739 B1**

(12) **FASCICULE DE BREVET EUROPEEN**

(45) Date de publication et mention
de la délivrance du brevet:
**19.07.2023 Bulletin 2023/29**

(21) Numéro de dépôt: **20753927.1**

(22) Date de dépôt: **06.08.2020**

(51) Classification Internationale des Brevets (IPC):
**G01T 1/164** (2006.01)    **G21K 1/04** (2006.01)

(52) Classification Coopérative des Brevets (CPC):
**G01T 1/1648; G21K 1/043**

(86) Numéro de dépôt international:
**PCT/EP2020/072208**

(87) Numéro de publication internationale:
**WO 2021/028327 (18.02.2021 Gazette 2021/07)**

(54) **COLLIMATEUR TOURNANT POUR UN SYSTÈME DE DÉTECTION DE RAYONS X**

ROTIERENDER KOLLIMATOR FÜR EIN RÖNTGENDETEKTIONSSYSTEM

ROTATING COLLIMATOR FOR AN X-RAY DETECTION SYSTEM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.08.2019 FR 1909114**

(43) Date de publication de la demande:
**15.06.2022 Bulletin 2022/24**

(73) Titulaires:
- **UNIVERSITE GRENOBLE ALPES**
  **38400 SAINT MARTIN D'HERES (FR)**
- **Surgiqual Institute**
  **38240 Meylan (FR)**
- **Centre Hospitalier Universitaire Grenoble Alpes**
  **38700 La Tronche (FR)**
- **Institut Polytechnique de Grenoble**
  **38000 Grenoble (FR)**
- **Centre national de la recherche scientifique**
  **75016 Paris (FR)**
- **UNIVERSITE CLAUDE BERNARD - LYON 1**
  **69100 Villeurbanne (FR)**
- **Ecole Centrale de Lyon**
  **69130 Ecully (FR)**
- **CPE Lyon Formation Continue et Recherche**
  **69100 Villeurbanne (FR)**

(72) Inventeurs:
- **GRONDIN, Yannick**
  **73800 Arbin (FR)**
- **CINQUIN, Philippe**
  **38330 Saint-Nazaire-les-Eymes (FR)**
- **DESBAT, Laurent**
  **38000 Grenoble (FR)**
- **GUIRAL, Pierrick**
  **69003 Lyon (FR)**
- **PITTET, Patrick**
  **69270 Fontaines-Saint-Martin (FR)**

(74) Mandataire: **Cabinet Chaillot**
**16/20, avenue de l'Agent Sarre**
**B.P. 74**
**92703 Colombes Cedex (FR)**

(56) Documents cités:
**FR-A1- 3 042 881    GB-A- 1 387 442**

- **A LEGRAND ET AL: "An ultrafast mechanical shutter for X-rays", NUCLEAR INSTRUMENTS AND METHODS IN PHYSICS RESEARCH SECTION A: ACCELERATORS, SPECTROMETERS, DETECTORS AND ASSOCIATED EQUIPMENT, vol. 275, no. 2, 15 février 1989 (1989-02-15), pages 442-446, XP055001570, ISSN: 0168-9002, DOI: 10.1016/0168-9002(89)90722-5**

EP 4 010 739 B1

**Description**

DOMAINE TECHNIQUE ET ART ANTÉRIEUR

La présente demande a trait au domaine des systèmes de détection de rayons X, et concerne plus particulièrement le domaine de la localisation spatiale d'éléments dans un système de détection de rayons X.

La présente demande s'applique en outre à l'imagerie par rayons X, notamment l'imagerie dite interventionnelle lors de laquelle, on cherche à déterminer précisément le positionnement d'un outil en même temps que l'on réalise une acquisition d'images, par exemple lors d'une intervention chirurgicale ou d'une opération réalisée à l'aide de cet outil.

Le document FR 2 841 118 présente un système d'imagerie par rayon X comprenant un moyen de détermination du positionnement d'un appareil de radiographie à l'aide d'une mire fixée sur un objet dont on acquiert une image.

Dans un tel système l'image de la mire interfère avec celle de l'objet que l'on souhaite étudier.

Le document US 6 490 475 présente quant à lui un système de fluoroscopie dans lequel on détermine le positionnement d'éléments du système à l'aide de marqueurs. Un traitement numérique est alors effectué pour pouvoir retirer ensuite l'empreinte de ces marqueurs de l'image finale.

Le document US 7 065 393 concerne un procédé de calibration d'un système d'imagerie par rayonnement X à l'aide de capteurs inertiels disposés sur différents éléments du système.

Ce type de capteur connaît souvent une large accumulation d'erreurs, une faible précision et peut donc poser des problèmes quant à la détermination fiable de la position d'un élément muni de capteurs dans un système d'imagerie par rayons X.

La demande de brevet FR3042881 présente un dispositif pour l'imagerie par rayons X comprenant un collimateur tournant, comprenant deux fentes. Un tel dispositif permet de déterminer de manière fiable la position dans un référentiel donné d'un élément muni de capteurs de rayons X destinés à recevoir des faisceaux de rayons X issus des fentes du collimateur.

La présente demande vise notamment à simplifier le dispositif de la demande de brevet FR3042881.

A cette fin, la présente demande concerne, selon un premier aspect, un dispositif de collimation pour un système de détection de rayons X, le dispositif de collimation comprenant:

- un collimateur comprenant un support sensiblement plan en matériau à radio transparence partielle ou nulle, le support étant mobile en rotation autour d'un axe de rotation traversant le support et perpendiculaire à une première face du support servant de plan d'incidence de rayons X appelé « plan principal du support », ledit support étant muni sur la première face d'une unique fente totalement transparente aux rayons X, configurée pour créer un flux de rayons X lorsque le collimateur est exposé à une source de rayons X, ladite fente s'étendant longitudinalement dans le plan principal du support selon un axe situé à une distance non nulle de l'axe de rotation, ladite fente s'étendant dans toute l'épaisseur du support.

Un tel collimateur à fente unique permet une réalisation simplifiée du dispositif de collimation.

Dans un ou plusieurs modes de réalisation, la fente présente une projection de forme rectangulaire ou trapézoïdale dans le plan principal du support.

Un autre objet de la présente demande concerne, selon un deuxième aspect, un système de détection de rayons X, le système de détection comprenant :

- une source de rayons X configurée pour émettre un faisceau de rayons X;
- un dispositif de collimation selon le premier aspect, placé de sorte à recevoir le faisceau de rayons X sur le plan d'incidence du support du dispositif de collimation ;
- un dispositif d'entraînement configuré pour mettre le support du dispositif de collimation en rotation autour de son axe avec une vitesse de rotation angulaire fixe ;
- au moins un élément de détection de rayons X placé dans un champ d'acquisition à l'opposé du support par rapport à la source de rayons X de sorte à acquérir des rayons X lorsqu'une partie du faisceau de rayons X traversant la fente du support de la première face du support à la face opposée, atteint l'élément de détection considéré au cours de la rotation du support.

Dans un ou plusieurs modes de réalisation, un élément de détection parmi ledit au moins un élément de détection de rayons X est configuré pour détecter des rayons X traversant la fente à deux instants successifs pendant que le support est en rotation, un premier instant correspondant à une première position angulaire de la fente et un deuxième instant correspondant à une deuxième position angulaire de la fente, les premières et deuxièmes positions angulaires définissant dans le plan principal du support un point d'intersection correspondant à la position projetée, dans le plan principal du support, de l'élément de détection, le système de détection comprenant une unité de traitement configurée pour déterminer la position projetée, dans le plan principal du support, de l'élément de détection à partir des première et deuxième positions angulaires. Ainsi chaque rotation (tour complet), on obtient deux instants successifs et donc deux positions angulaires. Les première et deuxième positions angulaires sont par exemple déterminées sur la base des deux instants successifs et de la vitesse de rotation angulaire du support.

Dans un autre mode de réalisation, la détermination des première et deuxième positions angulaires implique une fourche optique et le système de détection de rayons X comprend en outre un deuxième support, solidaire du support ou faisant partie intégrante du support, comprenant une série de N fenêtres uniformément distribuées angulairement sur un pourtour circulaire centré par rapport à l'axe de rotation du support, ladite série de N fenêtres comprenant une fenêtre de référence de taille différente des autres fenêtres, les autres fenêtres étant indexées par rapport à cette fenêtre de référence.

Dans ce cas, un élément de détection parmi ledit au moins un élément de détection de rayons X est configuré pour détecter des rayons X traversant la fente pour des première et deuxième positions angulaires du deuxième support définissant dans le plan principal du support un point d'intersection correspondant à la position projetée dans le plan principal du support de l'élément de détection. Dans ce mode de réalisation, le système de détection de rayons X comprend une fourche optique, la fourche optique étant doté d'un émetteur configuré pour émettre une onde lumineuse et un détecteur configuré pour détecter l'onde lumineuse, ladite fourche étant configurée pour générer des impulsions électriques lorsque, au passage d'une quelconque des fenêtres entre l'émetteur et le récepteur, le récepteur reçoit l'onde lumineuse, les première et deuxième positions angulaires correspondant chacune au passage d'une fenêtre. L'unité de traitement est alors configurée pour déterminer les première et deuxième positions angulaires sur la base des index des deux fenêtres (20b) ayant produit les impulsions électriques les plus proches temporellement des deux instants successifs pour lesquels la détection par l'élément de détection (DT) a eu lieu.

Dans un ou plusieurs modes de réalisation, l'unité de traitement est configurée pour déterminer la distance entre un plan passant par l'élément de détection et parallèle au plan principal du support et la source de rayons X à partir d'une durée d'irradiation de l'élément de détection, de la vitesse de rotation angulaire du support, de la distance entre la source de rayons X et le support, de la largeur de la fente, et de la distance entre l'intersection de l'axe de rotation du support et la position projetée dans le plan principal du support.

Dans un ou plusieurs modes de réalisation, la source de rayons X est une source pulsée configurée pour émettre des rayons X par cycles d'émission composés de deux modes utilisés alternativement, l'un étant un mode d'émission de rayons X, l'autre étant un mode de non émission de rayons X. Un élément de détection parmi le ou les éléments de détection de rayons X est alors configuré pour détecter des rayons X traversant la fente (30) de manière synchronisée avec les cycles d'émission des rayons X.

Dans un ou plusieurs modes de réalisation, dans le cas où la source de rayons X est une source pulsée, le support du dispositif de collimation selon le premier aspect de la présente demande est conçu dans un matériau scintillant aux rayons X. Le support est alors apte à émettre des photons de scintillation lorsque le support est exposé à la source de rayon X. Le système de détection selon le deuxième aspect de la présente demande comprend en outre une photodiode configurée pour détecter les photons de scintillation et émettre un photocourant résultant des photons de scintillation détectés de sorte à déclencher la détection et l'acquisition par l'élément de détection de rayons X traversant la fente du support.

Dans ce cas, l'unité de traitement peut-être configurée pour utiliser le photocourant émis par la photodiode pour asservir automatiquement la vitesse de rotation angulaire du support sur la durée des impulsions de la source pulsée. La vitesse de rotation est telle que le collimateur fait au moins un tour pendant la durée d'une impulsion.

La présente demande concerne, selon un troisième aspect, un système d'imagerie par rayons X comprenant un système de détection de rayons X selon le deuxième aspect, une pluralité d'éléments d'acquisition formant une matrice d'acquisition placée dans un champ d'acquisition du système d'imagerie, chaque élément d'acquisition de la matrice d'acquisition étant configuré pour acquérir une accumulation de flux de rayons X lorsque le support du collimateur du système de détection est en rotation autour de son axe, la matrice d'acquisition étant configurée pour acquérir une image radiographique à partir des acquisitions réalisées respectivement par les éléments d'acquisition constituant la matrice pendant une période de temps donnée.

Dans un ou plusieurs modes de réalisation, le système d'imagerie par rayons X comprend en outre un diaphragme plan placé entre la source de rayons X et le support tournant à fente unique, délimitant un champ de vue dont la projection sur la matrice d'acquisition ne dépasse pas la taille de la matrice d'acquisition. Ce diaphragme plan permet de ne pas illuminer d'autres zones que celle comprenant la pluralité d'éléments d'acquisition formant une matrice d'acquisition et limite spatialement le rayonnement de la source de rayons X, de sorte par exemple à ne pas illuminer un praticien ou opérateur utilisant le système d'imagerie par rayons X.

Dans un ou plusieurs modes de réalisation, la source de rayons X est une source pulsée configurée pour émettre des rayons X par cycles d'émission composés de deux modes utilisés alternativement, l'un étant un mode d'émission de rayons X, l'autre étant un mode de non émission de rayons X. Dans ce cas, la matrice d'acquisition est configurée pour acquérir des images radiographiques de manière synchronisée avec les cycles d'émission des rayons X.

Lorsque la source de rayons X est une source pulsée, le support est conçu dans un matériau scintillant aux rayons X, ledit support étant apte à émettre des photons de scintillation lorsque le support est exposé à la source de rayon X, ledit système d'imagerie comprenant alors en outre une photodiode configurée pour détecter les photons de scintillation et émettre un photocourant résultant des photons de scintillation détectés de sorte à déclencher l'acquisition par la matrice d'acquisition d'au moins une des images radiographiques.

Dans ce cas, l'unité de traitement peut être configurée pour utiliser le photocourant émis par la photodiode pour asservir automatiquement la vitesse de rotation angulaire du support sur la durée des impulsions de la source pulsée

La présente demande concerne en outre des procédés selon l'objet des revendications 16 à 23.

Selon un quatrième aspect, un procédé de localisation est défini par la revendication 17. Il permet la localisation d'un élément de détection d'un système de détection de rayons X selon le deuxième aspect.

Dans une ou plusieurs configurations, où le système de détection de rayons X selon le deuxième aspect comprend un deuxième support et une fourche optique, le procédé est défini par la revendication 18.

Dans un ou plusieurs modes de réalisation, le procédé de localisation selon le quatrième aspect comprend en outre une étape de détermination, par l'unité de traitement, de la distance entre un plan passant par l'élément de détection et parallèle au collimateur et la source de rayons X à partir d'une durée d'irradiation de l'élément de détection, de la vitesse de rotation angulaire du support, de la distance entre la source de rayons X et le support de la largeur de la fente.

Dans le cas où la source de rayons X est une source pulsée, et où le système de détection comprend une photodiode, l'unité de traitement utilise le photocourant émis par la photodiode pour asservir automatiquement la vitesse de rotation angulaire du support (V) sur la durée des impulsions de la source pulsée. Ceci permet de garantir une vitesse de rotation d'au moins 360° sur la durée d'une impulsion.

Un autre objet de la présente demande porte sur un procédé d'imagerie par rayons X mettant en oeuvre le système d'imagerie par rayons X selon le deuxième aspect, ledit procédé d'imagerie étant défini par la revendication 21.

Dans le cas où le procédé d'imagerie par rayons X est mis en oeuvre par un système d'imagerie par rayons X comprenant une source pulsée, et dans lequel le support du collimateur est conçu dans un matériau scintillant aux rayons X, et est apte à émettre des photons de scintillation lorsque le support est exposé à la source de rayon X, ledit système d'imagerie par rayons X comprenant en outre une photodiode configurée pour détecter les photons de scintillation et émettre un photocourant résultant des photons de scintillation détectés de sorte à déclencher l'acquisition par la matrice d'acquisition d'au moins une des images radiographiques, le procédé est défini par la revendication 22.

Dans ce cas, l'unité de traitement peut utiliser le photocourant émis par la photodiode pour asservir automatiquement la vitesse de rotation angulaire du support sur la durée des impulsions de la source pulsée de sorte que l'acquisition d'une image radiographique à partir des détections réalisées respectivement par les éléments d'acquisition de la matrice d'acquisition soit fenêtrée temporellement sur les modes d'émission de rayons X des cycles d'émission de la source pulsée. L'image peut être construite par accumulation des signaux détectés sur une ou plusieurs fenêtres temporelles.

D'autres avantages et particularités de la présente demande résulteront de la description qui va suivre, donnée à titre d'exemple non limitatif et faite en référence aux figures annexées :

[Fig. 1] représente un dispositif de collimation selon un mode de réalisation.

[Fig. 2] illustre la notion de radio transparence partielle avec des données quantitatives relatives à différents matériaux.

[Fig. 3] est une représentation simplifiée d'un système de détection de rayons X.

[Fig. 4] illustre des aspects du calcul d'une position projetée dans un plan d'un support d'un dispositif de collimation.

[Fig. 5] représente schématiquement un système de détection de rayons X selon un exemple de réalisation.

[Fig. 6A] est un schéma représentant le principe d'une fourche optique.

[Fig. 6B] illustre schématiquement le principe de fonctionnement de la boucle à verrouillage de phase

[Fig. 7] montre des résultats de mesure d'instants t_alpha et t_beta, pour trois positions différentes d'un élément de détection de rayons X placé dans un corps fantôme.

[Fig. 8] présente différents résultats de localisation d'un élément de détection obtenus par un mode de réalisation d'un procédé de localisation.

[Fig. 9] illustre l'irradiation d'un élément de détection par un flux issu de la fente d'un dispositif de collimation selon un exemple de réalisation.

[Fig. 10] représente de manière schématique un système d'imagerie par rayons X selon un exemple de réalisation.

[Fig. 11] illustre le balayage d'un champ de vue par une succession de flux de rayons X produits par rotation d'un dispositif de collimation selon un exemple de réalisation.

[Fig. 12] représente trois images radiographiques obtenues par un mode de réalisation d'un procédé d'imagerie par rayons X.

[Fig. 13] illustre un mode de réalisation d'une fente d'un dispositif de collimation selon un exemple de réalisation.

[Fig. 14A] et [Fig. 14B] illustre chacun un mode de réalisation d'un dispositif de collimation selon un exemple de réalisation permettant une calibration.

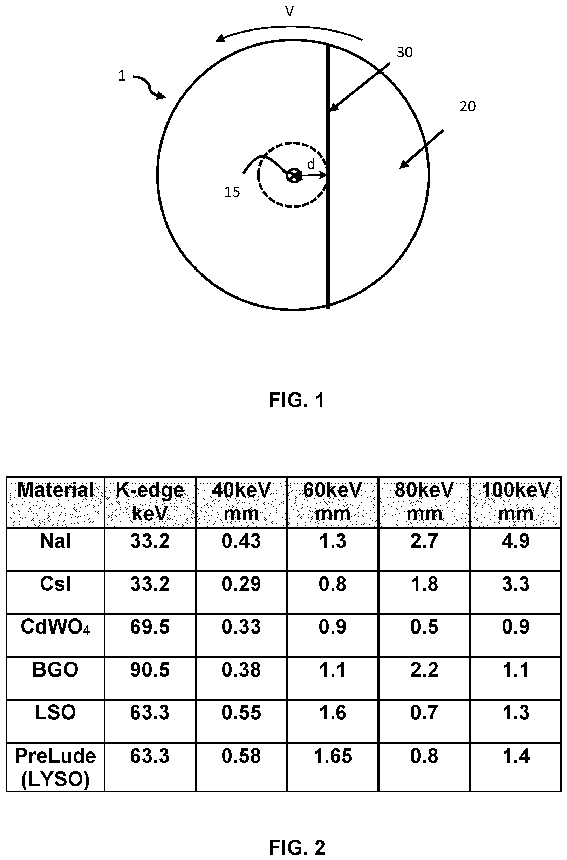

La figure 1 représente un dispositif de collimation 1 pour un système de détection de rayons X 2. Le dispositif de collimation 1 comprend un collimateur comprenant un support sensiblement plan 20 conçu dans un matériau à radio transparence partielle ou nulle. Sur la figure 1, le support 20 est représenté sous forme de disque, mais le support pourrait prendre toute autre forme bidimensionnelle. Le support 20 peut-être mobile en rotation autour d'un axe Δ 15 de rotation traversant le support 20 et perpendiculaire à une première face du support 20 servant de plan d'incidence de rayons X et appelé « plan principal du support » P. Le support 20 est muni d'une fente 30 s'étendant longitudinalement dans le plan principal du support P et dans toute l'épaisseur e du support 20. La fente 30 est totalement transparente aux rayons X et est située à une distance d non nulle de l'axe de rotation Δ 15. La fente peut être de forme rectangulaire, comme sur la figure 1. Dans ce cas, la fente présente une largeur l. La fente peut aussi être de forme trapézoïdale dans le plan principal du support.

Un dispositif de collimation (ou collimateur) permet de limiter spatialement un faisceau incident. Dans le contexte de l'application décrite dans ce document, le faisceau en amont du dispositif de collimation est par exemple de type « cone-beam » (faisceau conique) et le faisceau en aval du dispositif de collimation est de type « fanbeam » (faisceau en éventail). Le faisceau collimaté en sortie du dispositif de collimation balaie l'espace afin notamment de permettre la détermination de la position dans l'espace d'un élément de détection (par exemple, la position d'un capteur accroché à un outil qu'on essaie de localiser).

En pratique, la fente a une certaine épaisseur (correspondant à celle du support) et peut avoir une forme en 3D correspondant à un parallélépipède rectangle ou bien avoir une section transversale (dans le plan perpendiculaire au support) non rectangulaire. Par exemple, une inclinaison peut être prévue en fonction de la divergence du faisceau de rayons X émanant de la source de rayons X comme expliqué dans la demande de brevet FR3042881 au regard des figures 2A et 2B.

Par « radio transparence partielle», on entend un pourcentage non nul d'atténuation d'un flux de photons mono-énergétiques. Ce pourcentage dépend du matériau utilisé pour le support 20 et de l'épaisseur du support 20. La figure 2 montre différentes valeurs d'épaisseurs (en mm) de matériaux qui, pour une énergie de photons donnée (en keV) , permettent d'obtenir une atténuation de 95% d'un flux de photons mono-énergétiques incidents. Par exemple, un support 20 conçu en matériau $CdWO4$ d'épaisseur 0,9 mm permet d'atténuer de 95% le flux d'un faisceau de photons mono-énergétiques d'énergie 100 keV.

Par l'expression « totalement radiotransparente », on entend que la fente 30 ne présente pas d'absorption ou pas d'absorption significative du faisceau de rayons X. Par exemple, l'épaisseur ou le matériau, ou une combinaison de ces deux paramètres, de la fente définie dans le support peuvent être choisis pour obtenir une fente 30 totalement radiotransparente. Ce sera par exemple le cas, si la fente correspond à une ouverture dans le support (i.e. elle est remplie d'air).

Par « radio transparence nulle », on entend une opacité totale aux rayons X. Plus précisément, par « radio transparence nulle », on entend un pourcentage d'atténuation d'un flux de photons mono-énergétiques de plus de 99 pour cent. Par « radio transparence partielle », on entend un pourcentage d'atténuation d'un flux de photons mono-énergétiques compris entre 1 et 99 pour cent, par exemple supérieur à 10 pour cent, ou supérieur à 50 pour cent. Un pourcentage d'atténuation supérieur à 80 pour cent permet d'obtenir un bon compromis entre taux d'irradiation et qualité des images pouvant être acquises. Dans un tel cas, le collimateur absorbe fortement le faisceau de rayons X.

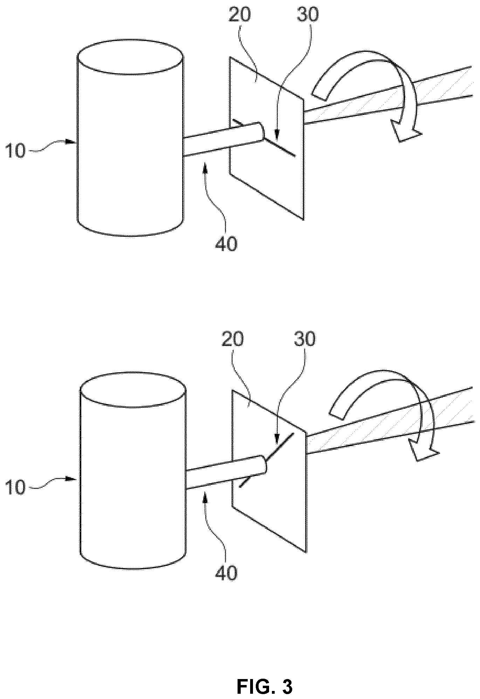

La figure 3 est une représentation d'un système de détection de rayons X 2. Le système de détection de rayons X 2 comprend :

- une source 10 de rayons X générant un faisceau de rayons X,

- un dispositif de collimation 1 selon l'un quelconque des modes de réalisation décrits dans la présente demande,
- un dispositif d'entraînement configuré pour mettre le support 20 du dispositif de collimation 1 en rotation autour de son axe de rotation Δ avec une vitesse angulaire fixe V,
- au moins un élément de détection de rayons X placé dans un champ d'acquisition à l'opposé du support 20 par rapport à la source 10 de rayons X.

L'élément de détection est placé de sorte à acquérir des rayons X lorsqu'un flux de rayons X (correspondant à une partie du faisceau généré par la source 10 de rayons X) traverse la fente 30, du plan d'incidence à la face opposée du support du dispositif de collimation, et atteint l'élément de détection considéré. La géométrie de ce flux de rayons X est dépendant de la forme de la fente 30. Par exemple, la géométrie du flux de rayons X peut être une géométrie en éventail. Par exemple, le flux de rayons X peut être un flux planaire.

La source 10 de rayons X est par exemple un tube à rayons X fonctionnant à des tensions entre électrodes se situant entre 40 et 125 kV.

Dans un premier mode de réalisation, lorsque le support 20 est entraîné en rotation par le dispositif d'entraînement, et que le dispositif de collimation 1 est exposé à des rayons X provenant de la source 10 de rayons X, lors d'une rotation du support 20, un élément de détection DT intercepte à deux instants, respectivement un premier instant t_alpha et un deuxième instant t_beta, un flux de rayons X de forte intensité créé par la fente 30. Le premier instant t_alpha correspond à une première position angulaire alpha et le deuxième instant t_beta correspond à une deuxième position angulaire beta. Les première et deuxième positions angulaires alpha et beta de la fente 30 définissent dans le plan principal du support P un point d'intersection C correspondant à la position projetée PT dans le plan principal du support 20 de l'élément de détection DT. Le point d'intersection C correspond également à l'intersection de la droite d'intersection des plans définis par les deux flux de rayons X interceptés par l'élément de détection DT avec le plan principal du support P.

Ainsi la détermination de la position du point d'intersection C est possible en utilisant un support tournant n'ayant qu'une seule fente, décentrée par rapport à l'axe de rotation du collimateur. Au cours d'une rotation du support, l'élément de détection DT est irradié 2 fois par le faisceau de rayons X traversant la fente selon 2 plans d'irradiation différents. L'intersection de ces deux plans est une droite reliant la source de rayons X à l'élément de détection DT. Il est ainsi possible de le localiser. L'hypothèse est faite ici que l'élément de détection DT ne bouge pas durant une rotation du support du fait que la vitesse de rotation du collimateur tournant est suffisamment grande (par exemple 1 tour par 25 ms) et que la vitesse de déplacement de l'élément de détection DT est suffisamment faible de sorte que le déplacement de l'élément de détection DT pendant un tour du collimateur soit négligeable.

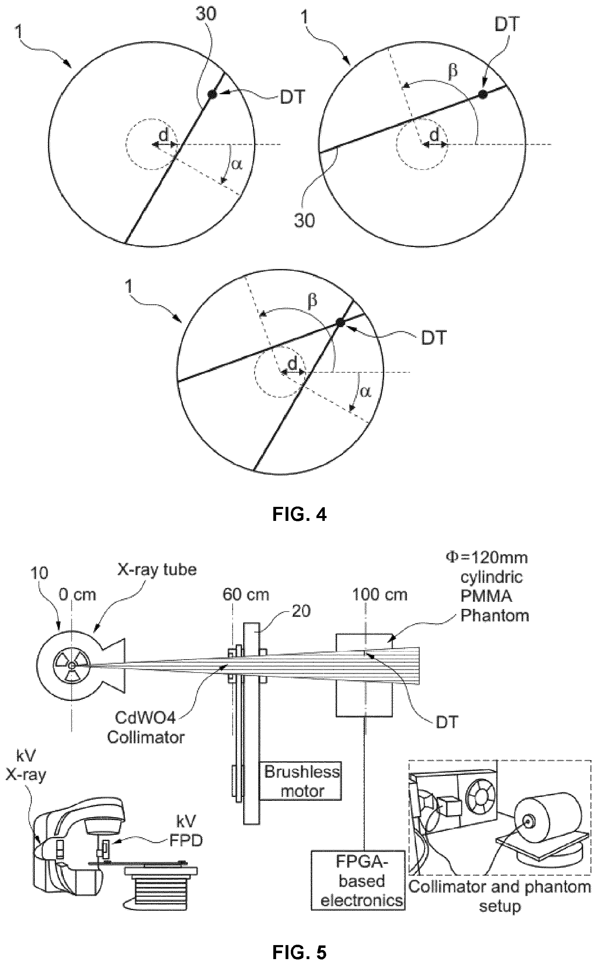

La figure 4 représente schématiquement les première et deuxième positions angulaires alpha et beta pour lesquelles l'élément de détection DT intercepte les flux de rayons X créés par la fente F aux premier et deuxième instants t_alpha et t_beta respectivement. La position alpha correspond au diagramme en haut à gauche de la figure 4 et la position beta correspond au diagramme en haut à droite de la figure 4. Le point d'intersection C correspondant à la position projetée PT dans le plan principal du support 20 de l'élément de détection DT est également représenté sur le digramme du bas de la figure 4.

Le système de détection de rayons X 2 peut en outre comprendre une unité de traitement 50 configurée pour déterminer la position projetée PT ou C dans le plan principal du support (P) de l'élément de détection (DT) sur la base des deux instants successifs (t_alpha) et (t_beta) déterminés pour l'élément de détection (DT) et de la vitesse de rotation angulaire du support V. En effet, en considérant un référentiel dans l'espace défini par un plan (xOy), avec O l'intersection de l'axe de rotation Δ avec le plan principal du support P, et un axe Oz égal à l'axe de rotation Δ, les coordonnées xc et yc de la position projetée de l'élément de détection DT dans le plan principal du support P sont les solutions du système d'équations :

[Math. 1]

$$x_C \cos alpha + y_C \sin alpha = d$$

[Math. 2]

$$x_C \cos beta + y_C \sin beta = d$$

où d est la distance entre la fente 30 et l'axe de rotation Δ, sin représente la fonction trigonométrique sinus et cos représente la fonction trigonométrique cosinus.

Ces deux équations forment un système linéaire qui a une solution unique. En effet, le déterminant de ce système linéaire est égal à *sin(beta-alpha)* est strictement positif, car les angles *alpha* et *beta* sont toujours différents du fait que la fente ne passe pas par l'intersection O entre le support 20 et l'axe de rotation Δ, et du fait que la différence diff=*beta-alpha* est toujours strictement inférieure ou égale à π.

Les angles *alpha* et *beta* peuvent être calculés à partir de la mesure des premier et deuxième instants t_alpha et t_beta, et de la connaissance de la vitesse de rotation angulaire V du support 20.

Les coordonnées xc et yc, autrement dit la position de la position projetée C dans le plan principal du support P de l'élément de détection DT, peuvent ainsi être déterminées sur la base des équations Math1 et Math2 à partir des angles *alpha* et *beta.* Leur connaissance permet de savoir que l'élément de détection DT se trouve sur la droite passant par la source 10 de rayons X et le point C de coordonnées xc et yc situé dans le plan principal du support P. L'élément de détection DT est ainsi, par la seule mesure des premier et deuxième instants t_alpha et t_beta, localisé de manière bidimensionnelle, dans le plan principal du support P. Cela permet, ensuite, de projeter la position de l'élément de détection DT dans un plan de formation d'images radiologiques, qui serait situé en aval de l'élément de détection DT, en utilisant une matrice de projection de l'ensemble formé par la source 10 de rayons X et le dispositif de collimation. Le formalisme mathématique de la projection d'un élément dans un tel plan de formation d'images peut être retrouvé dans la demande de brevet WO2017072125 (A1). Cette position projetée peut être utilisée soit pour des images radiologiques acquises antérieurement, soit en temps réel, dans le cas où des images radiologiques sont acquises simultanément avec la détection de l'élément DT et le calcul de cette position projetée.

La figure 5 représente schématiquement un système de détection 2 selon le deuxième aspect de la présente demande, où sont visibles une source 10 de rayons X sous la forme d'un tube à rayons X, un collimateur composé d'un support fabriqué en CdWO4, solidaire d'un moteur apte à entraîner le support 20 en rotation autour d'un axe de rotation, qui dans le cas de la figure 5, est horizontal, un élément de détection DT à base de nitrure de gallium (GaN) placé dans un corps fantôme cylindrique en polyméthacrylate de méthyle (PMMA), une unité de traitement 50 comprenant un circuit logique programmable de type FPGA (en anglais « field-programmable gâte array »).

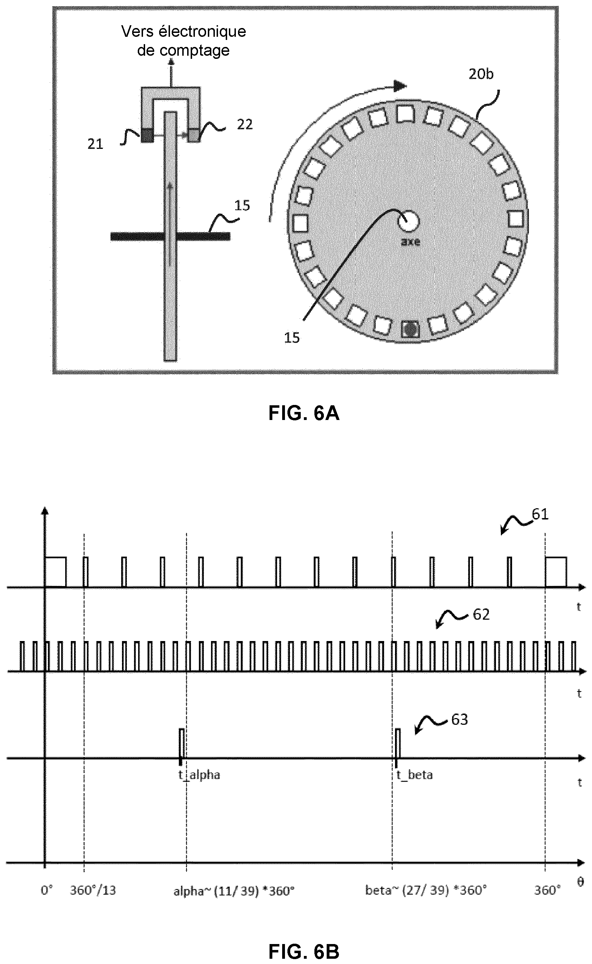

La figure 6A illustre schématiquement le principe de fonctionnement d'une fourche optique pour la mesure d'angles.

Dans un mode de réalisation, les angles alpha et beta peuvent être mesurés au moyen d'une fourche optique. Un deuxième support 20b peut être positionné parallèlement au plan principal du support P. Le deuxième support 20b est solidaire ou fait partie intégrante du support 20. Ce deuxième support 20b peut comprendre une série de N ouvertures (appelées dans la suite fenêtres), transparentes dans les domaines du visible ou du proche infra-rouge, uniformément distribuées angulairement sur un pourtour circulaire centré sur un point correspondant à l'intersection entre le deuxième support 20b et l'axe Δ de rotation 15. Les N fenêtres sont indexées par rapport à une fenêtre de référence, qui peut correspondre à une fenêtre plus large (ou respectivement plus étroite) que les autres fenêtres.

La fourche optique est dotée d'un émetteur 21 et d'un récepteur 22 en vis-à-vis. L'émetteur 21 émet une onde lumineuse, qui, lorsque cette onde lumineuse traverse une des fenêtres, est captée par le récepteur. Une impulsion électrique est produite ainsi lorsqu'une fenêtre laisse passer le signal de l'émetteur jusqu' au détecteur. L'impulsion électrique obtenue pour la fenêtre de référence sera plus large (ou respectivement plus étroite) que l'impulsion électrique reçue pour les autres fenêtres si cette fenêtre de référence est plus large (ou respectivement plus étroite).

Lorsque le dispositif de collimation 1 est exposé à des rayons X provenant de la source 10 de rayons X, lors d'une rotation du support 20, un élément de détection DT est exposé aux rayons X passant au travers de la fente 30 en une première position angulaire *alpha* et une deuxième position angulaire *beta.* Les impulsions électriques fournies par la fourche optique qui sont les plus proches temporellement des 2 instants de détection de DT permettent de déterminer directement les angles alpha et beta du support 20b et donc du support 20, en déterminant l'index des deux fenêtres correspondantes. L'index d'une fenêtre est déterminé par comptage du nombre d'impulsions détectées après l'impulsion de référence produite par la fenêtre de référence. Dans un mode de configuration avec N fenêtres, les angles *alpha* et *beta* peuvent être connus avec une précision en degrés de 360/N°. Ainsi, les coordonnées xC et yC de la position de la position projetée C dans le plan principal du support P de l'élément de détection DT peuvent être déterminées à partir des valeurs des positions angulaires *alpha* et *beta* mesurées. Cette configuration rend la détermination des angles *alpha* et *beta* plus robuste, car ce système de mesure est indépendant des variations possibles de la vitesse de rotation du support 20.

Dans une variante de ce mode de réalisation avec fourche optique, le deuxième support 20b peut comprendre une série avec un nombre limité de fenêtres indexées, par exemple 360 fenêtres indexées totalement. L'unité de traitement (50) comprend dans cette variante un système mettant en oeuvre une boucle à verrouillage de phase (de type PLL, Phase-Locked-Loop en anglais) qui permet d'obtenir une résolution angulaire supérieure à celle séparant deux fenêtres de détection successives. En effet, le système à verrouillage de phase fournit, un signal électrique à une fréquence multiple de la fréquence du signal électrique fourni par la fourche optique et en phase avec le signal électrique fourni par la fourche optique. Ainsi, entre deux fenêtres consécutives, espacées angulairement de 1°, l'unité de traitement 50 peut générer un signal dix fois plus rapide que la fréquence des impulsions électriques en sortie de la fourche optique. Cette configuration rend le système de mesure des angles *alpha* et *beta* plus précis en offrant une résolution angulaire augmentée.

La figure 6B illustre schématiquement le principe de fonctionnement de la boucle à verrouillage de phase. Le signal 61 est le signal fourni par la fourche optique (N=13). Le signal 62 est le signal fourni par le système à verrouillage de phase (signal en phase et de fréquence triple de celui fourni par la fourche optique, soit 39 impulsions). Le signal 63 est le signal fourni par un élément de détection DT qui comprend des impulsions aux instants t_alpha et t_beta. La détermination des angles alpha et beta du support 20 en découle comme illustré sur la dernière ligne en bas de la figure 6B: alpha est environ égal à (11/39)*360° et beta est environ égal à (27/39)*360°.

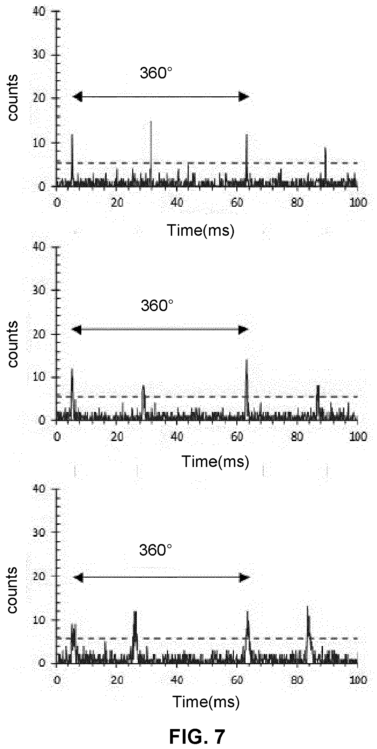

La figure 7 montre des résultats de mesure d'instants t_alpha et t_beta, pour trois positions différentes d'un élément de détection DT de rayons X placé dans un corps fantôme en PMMA, à des distances respectives de 4mm, 7mm et 12mm de l'axe de rotation Δ. L'écart entre les instants t_alpha et t_beta pour la distance de 4mm est plus grand que pour la distance de 12mm. En effet, plus l'élément de détection DT est proche de l'axe de rotation Δ, plus sa position projetée sur le plan principal du support P est proche de l'axe de rotation Δ, et plus la vitesse de la partie de la fente illuminant cette position projetée est lente.

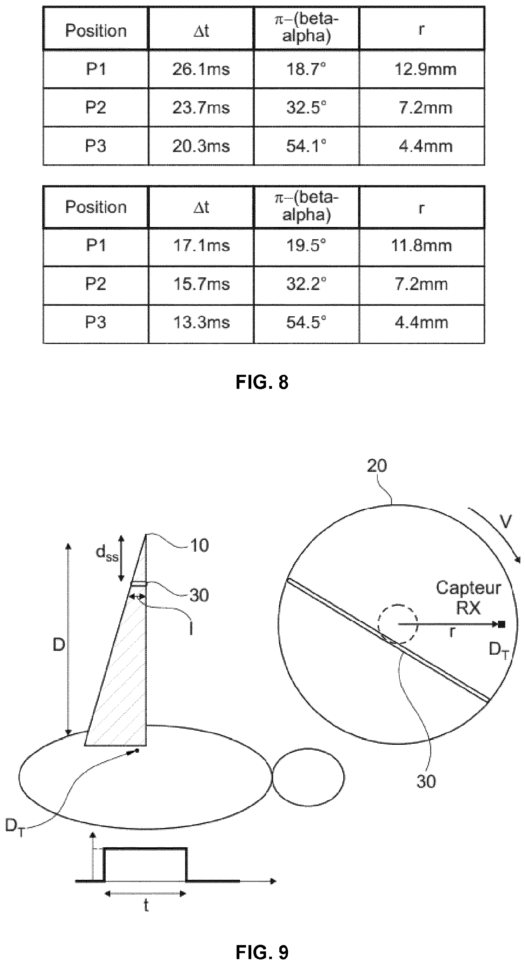

La figure 8 récapitule les différents résultats de localisation de l'élément de détection DT précédent obtenus par un mode de réalisation du procédé de localisation selon l'invention, mettant en oeuvre le système de détection précédemment décrit. Deux vitesses de rotation du support 20 ont été utilisées (1000 tours par minute pour le tableau du haut de la figure 8 et 1500 tours par minute respectivement pour le tableau du bas de la figure 8). Le nombre d'impulsions fluoroscopiques par seconde est de 60 et 38 respectivement. Les positions projetées P1, P2, P3 sont les positions déterminées respectivement pour 3 éléments de détection. Les mesures de la distance radiale r de l'élément de détection DT à l'axe de rotation Δ sont en concordance avec les distances réelles. Une précision millimétrique est obtenue.

Dans un autre aspect de la présente demande, l'unité de traitement 50 du système de détection de rayon X 2 précédemment décrit peut être aussi configurée pour déterminer la distance L entre le plan passant par l'élément de détection DT, et parallèle au collimateur, et la source 10 de rayons X à partir de la mesure d'une durée d'irradiation $t_{irr}$ de l'élément de détection DT, de la vitesse de rotation angulaire du support V, de la distance dss entre la source 10 de rayons X et le support 20 et de la largeur $l$ de la fente 30 La détermination de la distance L permet de localiser de manière tridimensionnelle, dans l'espace, l'élément de détection DT.

En faisant l'hypothèse que la source 10 de rayons X est divergente, il peut être en effet démontré que :

[Math. 5]

$$L = {t_{irr} r V d_{SS}}/{l}$$

où $t_{irr}$ représente la durée d'irradiation de l'élément de détection DT,
dss est la distance, connue par construction, entre la source 10 de rayons X et le support 20 et r est la distance entre la position projetée C dans le plan principal du support P et l'intersection de l'axe Δ avec le support 20. Cette formule utilise l'hypothèse d'une source 10 de rayons X divergente, permettant d'affirmer que plus l'élément de détection DT est loin de la source 10 de rayons X, plus la durée d'irradiation de l'élément de détection DT est longue.

La figure 9 illustre la propagation d'un flux planaire de rayons X issu de la fente 30 du support 20 irradiée par la source 10 de rayons X présentant une divergence, jusqu'à l'élément de détection $D_T$. Plus l'élément de détection DT est éloigné de la source 10 de rayons X, plus le signal reçu par l'élément de détection DT est élargi temporellement.

En particulier, on peut envisager que l'élément de détection DT est un détecteur de rayons X miniaturisé fixé sur un objet T mobile par rapport à la source 10 de rayons X, tel qu'un instrument chirurgical, ou un cathéter. Le système de détection 2 peut permettre dans ce cas à un praticien ou un opérateur de localiser en temps réel et dans trois dimensions un instrument chirurgical introduit dans le corps d'un patient. Pour cela, l'hypothèse est faite que la vitesse de rotation V du support 20 est très grande par rapport à la vitesse de déplacement de l'objet T tracé. Par exemple, l'élément de détection DT peut être une sonde de diamètre externe typiquement inférieur à 800 microns, comprenant un transducteur GaN radioluminescent couplé optiquement à une fibre optique, où la fibre optique est elle-même connectée à son autre extrémité à un module de photo-détection opéré en mode « comptage de photons ».

Dans un ou plusieurs modes de réalisation, la source 10 de rayons X est une source pulsée configurée pour émettre des rayons X par cycles d'émission composés de deux modes utilisés alternativement, l'un étant un mode d'émission de rayons X, l'autre étant un mode de non émission de rayons X. L'élément de détection DT est alors configuré pour détecter des rayons X traversant la fente 30 de manière synchronisée avec les cycles d'émission des rayons X. Dans ce cas, on peut en particulier choisir la vitesse de rotation du support 20 de sorte que le support 20 réalise un tour sur la durée du tir de la source pulsée, c'est-à-dire la durée du mode d'émission de rayons X. Cette durée d'émission peut durer de quelques millisecondes à quelques dizaines de millisecondes. Une vitesse angulaire V du support 20 est adaptée à 3000 tours par minute pour des durées d'émission de 20 millisecondes.

Par exemple, le support 20 peut être conçu dans un matériau scintillant aux rayons X, configuré pour émettre des photons de scintillation lorsque le support 20 est exposé à la source de rayon X. Le matériau scintillant peut être choisi parmi le CdWO4, le LSO, le LYSO et le BGO ou tout autre matériau de numéro atomique élevé. L'épaisseur du support 20 peut être comprise entre 0,5 et 4mm, selon l'atténuation de rayons X désirée. Dans ce cas, le système de détection 2 comprend en outre une photodiode PD configurée pour détecter les photons de scintillation et émettre un photocourant résultant des photons de scintillation détectés. Un exemple de photodiode PD pouvant être utilisée est le commutateur optique OPL551 commercialisé par la société Farnell. La détection des photons de scintillation par la photodiode permet de déclencher l'acquisition, par l'élément de détection DT, de rayons X traversant la fente 30. En d'autres termes, ne sont détectés que les rayons X pour lesquels la scintillation du support 20 est détectée. Ceci peut par exemple servir à soustraire un niveau de fond qui serait déterminé lorsque l'élément DT n'est pas irradié (i.e. en l'absence de scintillation du support) pour améliorer le contraste de détection et faciliter le choix du seuil de détection pour réduire les fausses détections.

Dans la configuration précédemment décrite, l'unité de traitement 50 peut utiliser le photocourant émis par la photodiode pour asservir automatiquement la vitesse de rotation angulaire du support V sur la durée des modes d'émission de la source pulsée. Ainsi, l'élément de détection DT détecte des rayons X seulement lorsque la source 10 de rayons X est en mode d'émission de rayons X, ce qui permet de s'affranchir du bruit existant en l'absence d'émission par la source 10 de rayons X.

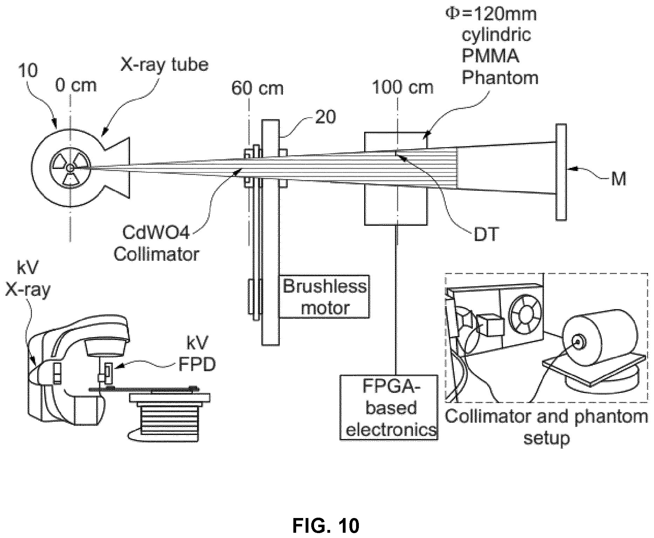

La figure 10 représente de manière schématique un système d'imagerie par rayons X 3. Ce système d'imagerie par rayons X 3 peut mettre en oeuvre le système de détection de rayons X 2 selon l'un quelconque des modes de réalisation décrits dans ce document. Un tel système d'imagerie 3 est destiné à créer des images radiographiques, notamment de parties corporelles d'un patient. Un tel système d'imagerie 3 comprend une pluralité d'éléments d'acquisition Mi formant une matrice d'acquisition M placée dans le champ d'acquisition du système d'imagerie 3. Chaque élément Mi de la matrice d'acquisition M peut acquérir, par intégration, un flux de rayons X, transmis à travers la fente et / ou à travers le support 20 lorsque le support 20 est choisi partiellement radio-transparent, lorsque le support 20 du collimateur du système de détection est en rotation autour de son axe Δ. Il est ainsi possible de reconstituer des images de 2 manières différentes : soit après une ou plusieurs rotations de la fente (balayage radial) une image fluoroscopique est obtenue pour, ou bien en utilisant un support dont la radio-opacité est ajustée de sorte à laisser passer suffisamment de rayons X pour pouvoir reconstruire une image, éventuellement dégradée. Ces deux modes d'acquisition d'image(s) dans un plan d'imagerie sont utilisables en combinaison avec la méthode de détection de position d'un élément de détection DT, placé en amont dans un autre plan, telle que décrite dans ce document. La qualité de l'image obtenue dépendra notamment du nombre de rotations du support utilisé pour l'acquisition de chaque image, du matériau utilisé pour le support et son épaisseur. En couplant l'un de ces modes d'acquisition d'image avec la méthode de détection de position d'un élément de détection DT, on peut en temps réel localiser l'élément de détection DT dans une image (éventuellement dégradée).

La matrice d'acquisition M peut ainsi acquérir une image radiographique à partir des acquisitions réalisées respectivement par les éléments d'acquisition Mi constituant la matrice d'acquisition M pendant une période de temps donnée. Il est à noter que la cadence de la solution d'imagerie présentée ici n'est pas suffisante pour un suivi d'un instrument chirurgical, tel un cathéter, mais permet une mise à jour lente d'images préopératoires. Un exemple de matrice d'acquisition M peut être le modèle Pixium 3040 de la société THALES, de dimension 293 x 398 mm, de résolution 1904 x 2586 pixels, et dont les pixels ont une taille de 154 microns.

Sur la figure 10, il peut être observé que le système d'imagerie 3 comprend un système de détection tel que précédemment décrit ainsi qu'une matrice d'acquisition M placée dans un plan d'imagerie en aval du corps fantôme. On peut voir que la matrice d'acquisition M est irradiée par le flux de rayons X ayant traversé le corps fantôme en PMMA.

Dans un ou plusieurs modes de réalisation, le système d'imagerie par rayons X 3 comprend un diaphragme plan 21 placé entre la source 10 de rayons X et le support 20, délimitant un champ de vue dont la projection FOV sur la matrice d'acquisition M ne dépasse pas la taille de la matrice d'acquisition M. Par exemple, pour un champ FOV carré de 30 cm de côté et un agrandissement de 3, un diaphragme plan 21 sera constitué de deux paires de « mâchoires » formant un champ carré de 10 cm de côté. Typiquement, la distance entre la source 10 de rayons X et la matrice d'acquisition M, placée derrière le patient mais à une distance relativement proche, est égale à 100 cm. Typiquement, la distance entre la source 10 de rayons X et le support 20 est de 40 cm. Typiquement, la distance entre la source 10 de rayons X et le diaphragme plan 21 est de 35 cm. Typiquement, la distance entre la source 10 de rayons X et un élément de détection DT introduit dans le corps d'un patient est de 80 cm. En général, le détecteur est de forme rectangulaire et le diaphragme est aussi rectangulaire. Dans ces modes de réalisation, l'ordre suivant le parcours des rayons X : source - diaphragme - collimateur tournant à fente unique - patient avec cathéter muni de d'un élément de détection DT - détecteur 2D de rayons X (par exemple, matrice de pixels) pour l'acquisition d'une ou plusieurs images.

Un tel système d'imagerie 3 permet non seulement de contrôler la dose d'irradiation des patients, mais aussi de coupler d'une part la production d'images radiographiques, et d'autre part la localisation tridimensionnelle, en temps réel, d'un élément chirurgical utilisé pour une opération médicale mettant en oeuvre un système d'imagerie par rayons X. Un tel élément est par exemple un cathéter inséré dans le corps d'un patient lors d'une intervention de cathétérisation.

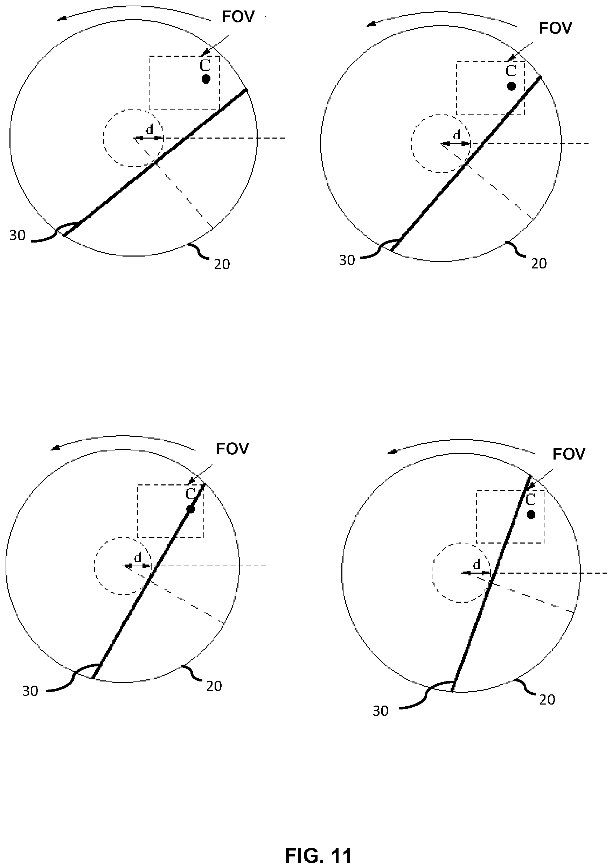

La figure 11 illustre plusieurs positions angulaires successives du support 20 et donc de la fente 30 obtenues par rotation du support 20 au cours du temps. Ainsi, la fente 30 crée un balayage d'irradiation de la projection FOV sur la matrice d'acquisition M, provenant de l'intersection à chaque instant entre le flux de rayons X issu de la fente 30 et la projection FOV sur la matrice d'acquisition M.

Dans le cas de l'utilisation d'une source pulsée, configurée pour émettre des rayons X par cycles d'émission composés de deux modes utilisés alternativement, l'un étant un mode d'émission de rayons X, l'autre étant un mode de non émission de rayons X, la matrice d'acquisition M est configurée pour acquérir des images radiographiques de manière synchronisée avec les cycles d'émission des rayons X.

En particulier, dans le cas où le support 20 est conçu dans un matériau scintillant aux rayons X, et peut émettre des photons de scintillation lorsqu'il est exposé à la source de rayon X, et dans le cas où le système de détection comprend en outre une photodiode PD qui peut détecter les photons de scintillation et émettre un photocourant résultant des photons de scintillation détectés, la détection des photons de scintillation par la photodiode permet de déclencher l'acquisition par la matrice d'acquisition M d'au moins une des images radiographiques. Ainsi, l'acquisition par la matrice d'acquisition M d'images radiographiques est fenêtrée, les fenêtres correspondant aux différents modes d'émission de rayons X par la source 10 de rayons X. Chaque image radiographique acquise par la matrice d'acquisition M provient d'une accumulation de rayons X sur plusieurs cycles d'émission de la source 10 de rayons X. Ceci permet à la matrice d'acquisition M de ne détecter que les rayons X provenant de la source 10 de rayons X, et de ne pas détecter un éventuel bruit pouvant dégrader la qualité des images radiographiques acquises par la matrice d'acquisition M.

Aussi, l'unité de traitement peut utiliser le photocourant émis par la photodiode PD pour asservir automatiquement la vitesse de rotation angulaire du support V sur la durée des impulsions de la source pulsée. Ceci permet de garantir que le support aura réalisé au moins un tour sur la durée d'une impulsion sans nécessiter d'interconnexion entre l'unité de traitement et le système de génération des rayons X.

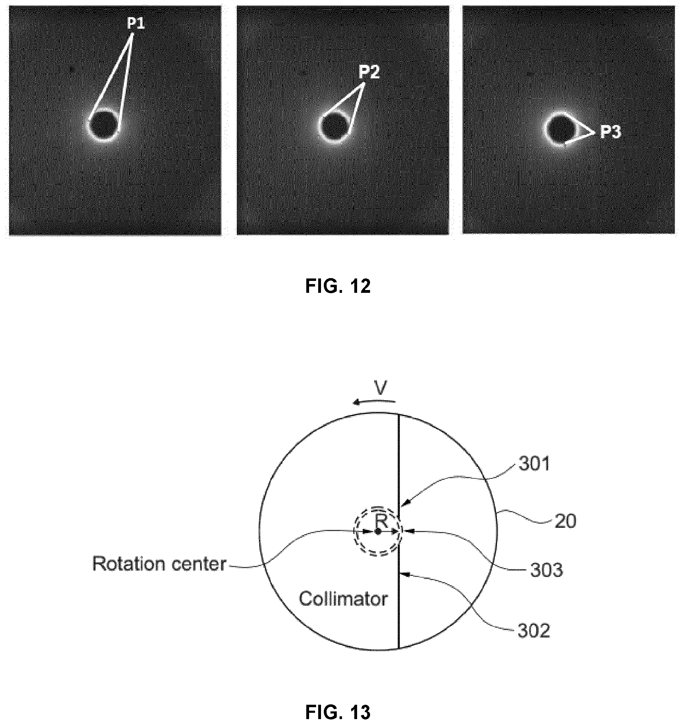

La figure 12 représente trois images radiographiques obtenues par un mode de réalisation du procédé d'imagerie par rayons X selon l'invention, mettant en oeuvre un système d'imagerie 3 tel que précédemment décrit. Chacune des images correspond à une position d'un élément de détection DT miniaturisé positionné, de gauche à droite sur la figure 12, respectivement à 12mm, 7mm et 4mm de l'axe de rotation Δ. Sur chacune des trois images, on peut observer au centre un disque noir. Ce disque noir est caractéristique de la distance d à laquelle est placée la fente 30 sur le support 20. Ce disque noir, ou zone morte, est entouré d'un halo très lumineux correspondant aux endroits de la fente où celle-ci est tangente au disque noir : en effet, en ces endroits, la vitesse de rotation du support 20 est la plus lente, ce qui a pour effet d'irradier plus longuement la fente 30. A delà de ce halo lumineux, la luminosité décroît vers la périphérie du support 20.

Ainsi, l'invention présente l'avantage de pouvoir coupler deux modes d'un système d'imagerie par rayons X 3 : un mode de localisation et de suivi « temps réel » d'objets, tels que des instruments chirurgicaux dans un corps, et un mode d'imagerie « basse cadence », permettant de créer des images radiographiques avec des doses de rayons X diminuées du fait de la faible radio transparence du dispositif de collimation selon l'invention. Un autre avantage réside dans l'utilisation d'un matériel simplifié (un collimateur à une seule fente).

Des améliorations du dispositif de collimation 2 et des systèmes de détection 2 et d'imagerie 3 ainsi que des procédés de localisation et d'imagerie associés sont possibles en optimisation de la géométrie de la fente, à la fois dans le plan principal du support P, et dans l'épaisseur du support 20, et ce, afin de réduire davantage la dose d'irradiation des patients et des praticiens.

Par exemple, une telle réduction pourrait être obtenue en atténuant le halo très lumineux visible en périphérie de la zone morte présente dans les images obtenues par le procédé d'imagerie selon l'invention et précédemment décrite.

Un mode de réalisation de fente 30 pour atténuer le halo lumineux et diminuer la dose d'irradiation pourrait être l'utilisation d'une fente 30 moins profonde, c'est à dire qui garde une épaisseur de matériau radio-opaque au voisinage de l'axe de rotation Δ du support 20, sur une portion 30a, et qui serait plus profonde, voire découpée dans le support 20, c'est-à-dire traversant le matériau du support 20 au voisinage du bord du support 20, sur une portion 30b.

Un autre mode de réalisation de fente 30 pour atténuer le halo lumineux et diminuer la dose d'irradiation serait de définir une fente 30 excentrée d'une distance d de l'axe de rotation Δ telle que décrite dans la présente demande par trois zones : deux segments de fente 301 et 302 distants d'une deuxième distance d' du point de tangence de la fente 30 avec un cercle de rayon d centré sur la projection de l'axe de rotation du support Δ sur le support 20, et une partie centrale 303 radio-opaque (ou moins radio-transparente).

La figure 13 illustre schématiquement un dispositif de collimation avec une telle fente 30. Sur la figure 13, un support 20 prenant la forme d'un disque est représenté. Le support 20 comprend une fente 30 excentrée d'une distance R par rapport à l'axe de rotation Δ constituée de deux segments de fente 301 et 302, distants chacun d'une deuxième

# EP 4 010 739 B1

distance dd' du point de tangence de la fente 30, et d'une partie centrale 303. Les deux segments de fente 301 et 302 présentent une radio transparence partielle. La partie centrale 303présente une radio transparence nulle.

Dans un mode de réalisation du dispositif de collimation, le plan principal du support, contenant la fente, peut en outre contenir un ou plusieurs trous ou canaux : ces canaux servent à réaliser la calibration du dispositif de collimation au sein d'un système d'imagerie selon l'un quelconque des modes de réalisation décrit dans ce document. Les canaux permettent de déterminer la matrice de projection du plan principal du support vers le plan d'acquisition des images (appelé aussi plan image). Ainsi la position d'un élément de détection, initialement calculée dans le plan principal du support du dispositif de collimation 1, peut être déterminée dans le plan image par application de cette matrice de projection.

Cette matrice de projection rassemble les informations géométriques de localisation du plan du support par rapport à la source et le détecteur de rayons X du système d'imagerie. Pour cela, avant de procéder à la phase de recherche de la position d'un élément de détection, on acquiert au moins une image sur laquelle figurera, non seulement la projection de la fente, mais également la projection de chaque canal, cette dernière étant formée par les rayons X traversant le canal considéré et parvenant au plan image.

Les propriétés des canaux peuvent être les suivantes. Le diamètre est choisi de sorte à ne pas trop irradier le patient mais suffisant pour laisser passer les rayons X afin de détecter de manière fiable la projection d'un canal dans le plan image. Le diamètre des canaux est par exemple du même ordre de grandeur que la largeur de la fente. Les canaux peuvent être positionnés en bordure du plan du support de la fente pour obtenir une meilleure précision de la calibration. La position de chacun des canaux dans le plan principal du support du dispositif de collimation est connue.

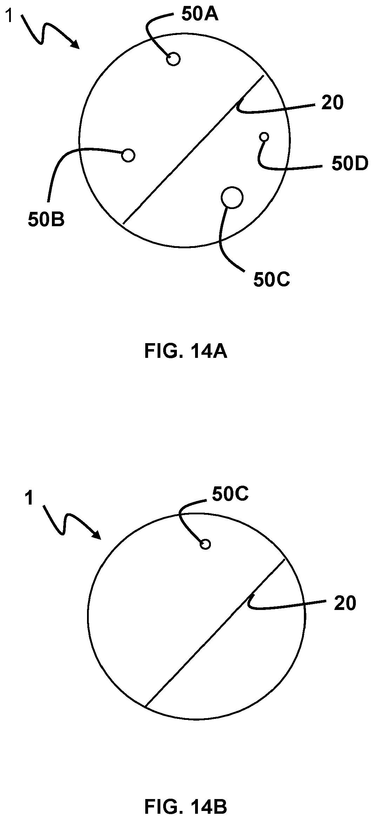

Lorsque le support comporte plusieurs canaux, les canaux doivent être distinguables entre eux. Pour cela, les canaux peuvent avoir des diamètres différents ou être positionnées de manière que l'arrangement formé permette de les distinguer. En outre, les canaux ne doivent pas être alignés : par exemple, si le plan principal du support comporte 4 canaux, ils doivent former un quadrilatère. La Fig. 14A illustre un mode de réalisation d'un dispositif de collimation 1 à fente unique 20 avec 4 canaux 50A, 50B, 50C, 50D de diamètres distincts.

Dans un autre mode de réalisation illustré à la figure 14B, le plan du support contenant la fente 20, contient un seul canal 50E avec les propriétés décrites précédemment. Comme le plan peut être mis en rotation, on peut simuler la présence d'au moins 3 autres canaux en faisant tourner le dispositif de collimation d'un angle connu à chaque fois et en acquérant une image pour chaque angle. Ce mode de réalisation a plusieurs avantages. Il y a moins de canaux dans le plan et par conséquent moins de rayons X pouvant atteindre le patient lors de la phase de localisation de l'élément de détection. Le problème de distinction de la projection des canaux sur le plan image ne se pose plus : on acquiert chaque image après rotation du support du dispositif de collimation. Chaque nouvelle position du canal dans le plan principal du support, qui est connue, est donc associée à sa position projetée dans le plan image. Comme uniquement le plan du support effectue une rotation, et qu'aucun autre élément ne bouge, la calibration de l'ensemble peut être effectuée.

La méthode de calibration utilisable dans ce contexte en vue de la détermination de la matrice de projection du plan du support vers le plan d'acquisition des images peut être celle décrite dans la demande de brevet FR3028039A1 ou WO2016071645A1. Dans cette méthode de calibration, on détermine la position dans le plan image des projections d'au moins N canaux à partir d'une ou plusieurs images acquises. On obtient la matrice de calibration intrinsèque du système « imagerie + collimateur » dans une position de référence et la position projetée des canaux dans le plan image pour cette position de référence. On calcule ensuite une homographie entre la position projetée des canaux dans les positions de référence et de mesure, en mettant en correspondance chaque canal. Pour cela, il est fait l'hypothèse qu'entre la position de référence et la position de mesure, l'ensemble « source + collimateur » est rigidement lié et donc ne bouge pas l'un par rapport à l'autre. Uniquement le détecteur image est autorisé à bouger librement relativement à l'ensemble « source + collimateur » entre les positions de référence et de mesure. Le nombre N de canaux utilisés pour la calibration doit être suffisant pour déterminer l'homographie. Par exemple, les canaux sont au moins au nombre de 4 (N=4). Dans le cas où l'ensemble « source + collimateur » n'est pas rigidement lié, d'autres méthodes sont utilisées pour projeter la position de l'élément de détection exprimée dans le plan du support vers le plan image. Par exemple, la matrice de calibration intrinsèque du système d'imagerie dans la position de mesure est obtenue par une calibration off-line à l'aide d'une mire 3D connue et en faisant l'hypothèse que le système d'imagerie est mécaniquement reproductible dans un intervalle de temps connu. Plus de détails sont donnés dans la demande de brevet WO2016071645A1.

**Revendications**

1. Dispositif de collimation (1) pour un système de détection de rayons X, le dispositif de collimation (1) comprenant:

un collimateur comprenant un support sensiblement plan (20) en matériau à radio transparence partielle ou nulle, le support (20) étant mobile en rotation autour d'un axe (Δ) de rotation traversant le support et perpendiculaire à une première face du support (20) servant de plan d'incidence de rayons X, appelé plan principal du support (P),

ledit support (D) étant muni sur la première face d'une unique fente (30) totalement transparente aux rayons X, configurée pour créer un flux de rayons X lorsque le collimateur est exposé à une source (10) de rayons X, ladite fente (30) s'étendant longitudinalement dans le plan principal du support selon un axe situé à une distance (d) non nulle de l'axe de rotation (Δ), ladite fente (30) s'étendant dans toute l'épaisseur du support (20).

2. Dispositif de collimation (1) selon la revendication 1 dans lequel la fente (30) présente une projection de forme rectangulaire ou trapézoïdale dans le plan principal du support (P).

3. Système de détection (2) de rayons X, le système de détection (2) comprenant

- une source (10) de rayons X configurée pour émettre un faisceau de rayons X;
- un dispositif de collimation (1) selon l'une quelconque des revendications précédentes, placé de sorte à recevoir le faisceau de rayons X sur le plan d'incidence du support ;
- un dispositif d'entraînement configuré pour mettre le support (20) du dispositif de collimation en rotation autour de son axe (Δ) avec une vitesse de rotation angulaire fixe (V) ;
- au moins un élément de détection de rayons X placé dans un champ d'acquisition à l'opposé du support (20) par rapport à la source (10) de rayons X de sorte à acquérir des rayons X lorsqu'une partie du faisceau de rayons X, traversant la fente (30) du support (20) de la première face du support à la face opposée, atteint l'élément de détection considéré au cours de la rotation du support.

4. Système de détection (2) selon la revendication 3,

dans lequel un élément de détection (DT) parmi ledit au moins un élément de détection de rayons X est configuré pour détecter des rayons X traversant la fente (30) à deux instants successifs pendant que le support (20) est en rotation, un premier instant (t_alpha) correspondant à une première position angulaire (alpha) de la fente et un deuxième instant (t_beta) correspondant à une deuxième position angulaire (beta) de la fente (30), les premières et deuxièmes positions angulaires définissant dans le plan principal du support ( P) un point d'intersection (C) correspondant à la position projetée (PT) dans le plan principal du support (20) de l'élément de détection (DT);

le système de détection (2)comprenant une unité de traitement (50) configurée pour déterminer la position projetée (C) dans le plan principal du support (P) de l'élément de détection (DT) à partir des première et deuxième positions angulaires.

5. Système de détection selon la revendication 4, l'unité de traitement étant configurée pour déterminer les première et deuxième positions angulaires sur la base des premier et deuxième instants et de la vitesse angulaire du support.

6. Système de détection (2)selon la revendication 4, comprenant en outre

- un deuxième support (20b) solidaire du support (20) ou faisant partie intégrante du support (20), comprenant une série de N fenêtres uniformément distribuées angulairement sur un pourtour circulaire centré par rapport à l'axe (Δ) de rotation du support, ladite série de N fenêtres comprenant une fenêtre de référence de taille différente des autres fenêtres, les autres fenêtres étant indexées par rapport à cette fenêtre de référence,
- une fourche optique, la fourche optique étant doté d'un émetteur configuré pour émettre une onde lumineuse et un détecteur configuré pour détecter l'onde lumineuse, ladite fourche étant configurée pour générer des impulsions électriques lorsque, au passage d'une quelconque des fenêtres entre l'émetteur et le récepteur, le récepteur reçoit l'onde lumineuse,

l'unité de traitement (50) étant configurée pour déterminer les première et deuxième positions angulaires sur la base des index des deux fenêtres (20b) ayant produit les impulsions électriques les plus proches temporellement des deux instants successifs pour lesquels la détection par l'élément de détection (DT) a eu lieu.

7. Système de détection (2) selon l'une quelconque des revendications 4 à 6, dans lequel l'unité de traitement (50) est configurée pour :

- déterminer la distance entre un plan passant par l'élément de détection (DT) et parallèle au plan principal du support (P) et la source (10) de rayons X, dans le cas où la fente est de forme rectangulaire, à partir d'une durée d'irradiation de l'élément de détection (DT), de la vitesse de rotation angulaire du support (V), de la distance (D) entre la source (10) de rayons X et le support (20) et de la largeur de la fente (I) et de la distance r entre l'intersection de l'axe de rotation (Δ) du support (20) et la position projetée (C) dans le plan principal (P) du support (20).

8. Système de détection (2) selon l'une quelconque des revendications 4 à 7, dans lequel :

   - la source (10) de rayons X est une source pulsée configurée pour émettre des rayons X par cycles d'émission composés de deux modes utilisés alternativement, l'un étant un mode d'émission de rayons X, l'autre étant un mode de non émission de rayons X
   - l'élément de détection (DT) est configuré pour détecter des rayons X traversant la fente (30) de manière synchronisée avec les cycles d'émission des rayons X.

9. Système de détection (2) selon la revendication 8, dans lequel le support (20) est conçu dans un matériau scintillant aux rayons X, ledit support (20) étant apte à émettre des photons de scintillation lorsque le support est exposé à la source de rayon X,
   ledit système de détection (2) comprenant en outre une photodiode (PD) configurée pour détecter les photons de scintillation et émettre un photocourant résultant des photons de scintillation détectés de sorte à déclencher la détection et l'acquisition par l'élément de détection (DT) de rayons X traversant la fente (30).

10. Système de détection (2) selon la revendication 9 dans lequel l'unité de traitement (50) est configurée pour utiliser le photocourant émis par la photodiode (PD) pour asservir automatiquement la vitesse de rotation angulaire du support (V) sur la durée des impulsions de la source pulsée.

11. Système d'imagerie par rayons X (3) comprenant :

    un système de détection (2) selon l'une quelconque des revendications 4 à 10 ;
    une pluralité d'éléments d'acquisition formant une matrice d'acquisition (M) placée dans un champ d'acquisition du système d'imagerie, chaque élément de la matrice d'acquisition (M) étant configuré pour acquérir une accumulation de flux de rayons X lorsque le support (20) du collimateur du système de détection est en rotation autour de son axe (Δ), la matrice d'acquisition (M) étant configurée pour acquérir une image radiographique à partir des acquisitions réalisées respectivement par les éléments d'acquisition constituant la matrice d'acquisition (M) pendant une période de temps donnée.

12. Système d'imagerie par rayons X (3) selon la revendication 11, comprenant en outre :

    un diaphragme plan (21) placé entre la source (10) de rayons X et le support (20),
    délimitant un champ de vue dont la projection sur la matrice d'acquisition (M) ne dépasse pas la taille de la matrice d'acquisition (M).

13. Système d'imagerie par rayons X (3) selon l'une quelconque des revendications 11 à 12 mettant en oeuvre un système de détection (2) suivant la revendication 8, dans lequel :
    la matrice d'acquisition (M) est configurée pour acquérir des images radiographiques de manière synchronisée avec les cycles d'émission des rayons X.

14. Système d'imagerie par rayons X (3) selon la revendication 13, mettant en oeuvre un système de détection (2) suivant la revendication 9, dans lequel la photodiode (PD) est configurée pour détecter les photons de scintillation et émettre un photocourant résultant des photons de scintillation détectés de sorte à déclencher l'acquisition par la matrice d'acquisition (M) d'au moins une des images radiographiques.

15. Système d'imagerie par rayons X (3) selon la revendication 14, mettant en oeuvre un système de détection (2) suivant la revendication 10 dans lequel l'unité de traitement (50) est configurée pour utiliser le photocourant émis par la photodiode (PD) pour asservir automatiquement la vitesse de rotation angulaire du support (V) sur la durée des impulsions de la source pulsée.

16. Procédé mis en oeuvre au moyen d'un système selon l'une quelconque des revendications 3 à 15, le procédé comprenant une étape de détection par au moins un élément de détection (DT) de rayons X traversant la fente (30) du support du dispositif de collimation lorsque le support est en rotation autour de son axe (Δ) et exposé à un faisceau de rayons X issus de la source.

**17.** Procédé selon la revendication 16, dans lequel le système est un système de détection de rayons X selon l'une quelconque des revendications 4 à 10, le support étant en rotation à une vitesse de rotation angulaire fixe ;

l'étape de détection comprenant une détection par un élément de détection (DT) de rayons X traversant la fente (30) du support à deux instants successifs, un premier instant (t_alpha) correspondant à une première position angulaire (alpha) de la fente et un deuxième instant (t_beta) correspondant à une deuxième position angulaire (alpha) de la fente ;

le procédé comprenant en outre une détermination par l'unité de traitement (50) de la position projetée (C) dans le plan principal (P) du support (20) de l'élément de détection (DT) sur la base des première et deuxième positions angulaires, les première et deuxième positions angulaires étant déterminées sur la base des premier et deuxième instants et de la vitesse angulaire du support.

**18.** Procédé selon la revendication 16, dans lequel le système est un système de détection de rayons X selon la revendication 6, le support étant en rotation à une vitesse de rotation angulaire fixe, le procédé comprenant :

- une détection d'impulsions électriques produites lorsque, au passage d'une quelconque des fenêtres entre l'émetteur et le récepteur de la fourche optique, le récepteur reçoit l'onde lumineuse;

l'étape de détection comprenant une détection par un élément de détection (DT) de rayons X traversant la fente (30) du support (20) pour une première position angulaire (alpha) du deuxième support (20b) et une deuxième position angulaire (beta) du deuxième support (20b),

le procédé comprenant en outre une détermination par l'unité de traitement (50) de la position projetée (C) dans le plan principal du support sur la base des première et deuxième positions angulaires,

les première et deuxième positions angulaires étant obtenues sur la base des index des deux fenêtres ayant produit les impulsions électriques les plus proches temporellement des deux instants successifs pour lesquels la détection par l'élément de détection (DT) a eu lieu.

**19.** Procédé selon la revendication 18, le système étant un système de détection (2) selon la revendication 10, dans lequel l'unité de traitement (50) utilise le photocourant émis par la photodiode (PD) pour asservir automatiquement la vitesse de rotation angulaire du support (V) sur la durée des impulsions de la source pulsée.

**20.** Procédé selon l'une quelconque des revendications 17 à 19, comprenant une étape de détermination, par l'unité de traitement (50), de la distance (D) entre un plan contenant l'élément de détection (DT) et parallèle au plan principal (P) du support (20) et la source (10) de rayons X à partir d'une durée d'irradiation (tirr) de l'élément de détection (DT), de la vitesse de rotation angulaire du support (V), de la distance (D) entre la source (10) de rayons X et le support (20) de la largeur de la fente (l)) et de la distance r entre l'intersection de l'axe de rotation (Δ) du support (20) et la position projetée (C) dans le plan principal (P) du support (20).

**21.** Procédé selon la revendication 16, le système étant un système d'imagerie par rayons X (3) selon l'une quelconque des revendications 11 à 12, le support étant en rotation à une vitesse de rotation angulaire fixe, le procédé comprenant les étapes suivantes :

- une détection de rayons X par les éléments d'acquisition de la matrice d'acquisition pendant la rotation du support (20) ;
- une acquisition d'une image radiographique à partir des détections réalisées respectivement par les éléments d'acquisition de la matrice (M) de détection pendant une période de temps donnée.

**22.** Procédé selon la revendication 16, le système étant un système d'imagerie par rayons X (3) selon la revendication 14, le support étant en rotation à une vitesse de rotation angulaire fixe, le procédé comprenant les étapes suivantes :

- détection de rayons X par les éléments d'acquisition de la matrice d'acquisition pendant la rotation du support (20) ;
- émission de photons de scintillation par le support (20) ;
- détection des photons de scintillation par la photodiode (PD),
- émission par la photodiode d'un photocourant résultant des photons de scintillation détectés de sorte à déclencher l'acquisition par la matrice d'acquisition (M) d'au moins une des images radiographiques,

- acquisition d'une image radiographique à partir des détections réalisées respectivement par les éléments d'acquisition de la matrice (M) de détection pendant une période de temps donnée.

23. Procédé selon la revendication 22, le système étant un système d'imagerie par rayons X (3) selon la revendication 15, dans lequel l'unité de traitement (50) utilise le photocourant émis par la photodiode (PD) pour asservir automatiquement la vitesse de rotation angulaire du support (V) sur la durée des impulsions de la source pulsée de sorte que l'acquisition d'une image radiographique à partir des détections réalisées respectivement par les éléments d'acquisition de la matrice (M) d'acquisition soit fenêtrée temporellement sur les modes d'émission de rayons X des cycles d'émission de la source pulsée.

**Patentansprüche**

1. Kollimationsvorrichtung (1) für ein Röntgenstrahlen-Detektionssystem, wobei die Kollimationsvorrichtung (1) umfasst:
einen Kollimator, der einen etwa ebenen Träger (20) aus einem Material umfasst, dessen Strahlentransparenz teilweise oder null ist, wobei der Träger (20) um eine Drehachse (Δ), die den Träger durchquert und rechtwinklig zu einer ersten Fläche des Trägers (20) ist, die als Einfallebene für die Röntgenstrahlen dient, bezeichnet als Trägerhauptebene (P), drehbeweglich ist, wobei der Träger (D) auf der ersten Fläche mit einem einzigen Schlitz (30), der für Röntgenstrahlen vollständig durchlässig ist, versehen ist, der dazu ausgelegt ist, einen Röntgenstrahlenstrom zu erzeugen, wenn der Kollimator einer Röntgenstrahlenquelle (10) ausgesetzt ist, wobei sich der Schlitz (30) längs in der Trägerhauptebene gemäß einer Achse erstreckt, die sich in einem Abstand (d) von nicht null von der Drehachse (Δ) befindet, wobei sich der Schlitz (30) in der gesamten Dicke des Trägers (20) erstreckt.

2. Kollimationsvorrichtung (1) nach Anspruch 1, wobei der Schlitz (30) eine rechteckige oder trapezförmige Projektion in der Trägerhauptebene (P) aufweist.

3. Röntgenstrahlen-Detektionssystem (2), wobei das Detektionssystem (2) umfasst:

   - eine Röntgenstrahlenquelle (10), die dazu ausgelegt ist, ein Röntgenstrahlenbündel zu senden;
   - eine Kollimationsvorrichtung (1) nach einem der vorangehenden Ansprüche, die derart platziert ist, dass sie das Röntgenstrahlenbündel auf der Einfallebene des Trägers empfängt;
   - eine Antriebsvorrichtung, die dazu ausgelegt ist, den Träger (20) der Kollimationsvorrichtung um seine Achse (Δ) mit einer festen Winkeldrehgeschwindigkeit (V) in Drehung zu versetzen;
   - mindestens ein Röntgenstrahlen-Detektionselement, das in einem Erfassungsfeld gegenüber dem Träger (20) im Verhältnis zu der Röntgenstrahlenquelle (10) derart platziert ist, dass Röntgenstrahlen erfasst werden, wenn ein Teil des Röntgenstrahlenbündels, das den Schlitz (30) des Trägers (20) von der ersten Fläche des Trägers auf die gegenüberliegende Fläche durchquert, das entsprechende Detektionselement während der Drehung des Trägers erreicht.

4. Detektionssystem (2) nach Anspruch 3,

   wobei ein Detektionselement (DT) von dem mindestens einen Röntgenstrahlen-Detektionselement dazu ausgelegt ist, Röntgenstrahlen zu detektieren, die den Schlitz (30) zu zwei aufeinanderfolgenden Momenten durchqueren, währenddessen sich der Träger (20) in Drehung befindet, wobei ein erster Moment (t_alpha) einer ersten Winkelposition (alpha) des Schlitzes entspricht und ein zweiter Moment (t_beta) einer zweiten Winkelposition (beta) des Schlitzes (30) entspricht, wobei die erste und zweite Winkelposition in der Trägerhauptebene (P) einen Schnittpunkt (C) definieren, der der projizierten Position (PT) in der Hauptebene des Trägers (20) des Detektionselements (DT) entspricht;
   wobei das Detektionssystem (2) eine Verarbeitungseinheit (50) umfasst, die dazu ausgelegt ist, die projizierte Position (C) in der Trägerhauptebene (P) des Detektionselements (DT) ausgehend von der ersten und zweiten Winkelposition zu bestimmen.

5. Detektionssystem nach Anspruch 4, wobei die Verarbeitungseinheit dazu ausgelegt ist, die erste und zweite Winkelposition auf der Basis des ersten und zweiten Moments und der Winkelgeschwindigkeit des Trägers zu bestimmen.

6. Detektionssystem (2) nach Anspruch 4, das ferner umfasst:

- einen zweiten Träger (20b), der mit dem Träger (20) fest verbunden ist oder fester Bestandteil des Trägers (20) ist, umfassend eine Reihe von N Fenstern, die gleichförmig winklig auf einem kreisrunden Umfang verteilt sind, der im Verhältnis zur Drehachse (Δ) des Trägers zentriert ist, wobei die Reihe von N Fenstern ein Referenzfenster unterschiedlicher Größe zu den anderen Fenstern umfasst, wobei die anderen Fenster im Verhältnis zu diesem Referenzfenster indexiert sind,

- eine optische Gabel, wobei die optische Gabel mit einem Sender versehen ist, der dazu ausgelegt ist, eine Lichtwelle zu senden, und einen Detektor, der dazu ausgelegt ist, die Lichtwelle zu detektieren, wobei die Gabel dazu ausgelegt ist, elektrische Impulse zu erzeugen, wenn beim Durchgang durch eines der Fenster zwischen dem Sender und dem Empfänger der Empfänger die Lichtwelle empfängt,

wobei die Verarbeitungseinheit (50) dazu ausgelegt ist, die erste und zweite Winkelposition auf der Basis der Indexe der zwei Fenster (20b) zu bestimmen, die die elektrischen Impulse erzeugt haben, die zeitlich den beiden aufeinanderfolgenden Momenten am nächsten sind, für die die Detektion durch das Detektionselement (DT) stattgefunden hatte.

7. Detektionssystem (2) nach einem der Ansprüche 4 bis 6, wobei die Verarbeitungseinheit (50) ausgelegt ist, um:

- den Abstand zwischen einer Ebene, die durch das Detektionselement (DT) verläuft und parallel zur Trägerhauptebene (P) ist, und der Röntgenstrahlenquelle (10), wenn der Schlitz rechteckig ist, ausgehend von einer Bestrahlungsdauer des Detektionselements (DT), von der Winkeldrehgeschwindigkeit des Trägers (V), vom Abstand (D) zwischen der Röntgenstrahlenquelle (10) und dem Träger (20) und von der Breite des Schlitzes (1) und vom Abstand r zwischen dem Schnitt der Drehachse (Δ) des Trägers (20) und der projizierten Position (C) in der Hauptebene (P) des Trägers (20) zu bestimmen.

8. Detektionssystem (2) nach einem der Ansprüche 4 bis 7, wobei:

- die Röntgenstrahlenquelle (10) eine gepulste Quelle ist, die dazu ausgelegt ist, Röntgenstrahlen in Sendezyklen zu senden, die aus zwei Modi zusammengesetzt sind, die alternativ genutzt werden, wobei einer ein Sendemodus von Röntgenstrahlen ist, wobei der andere ein Nichtsendemodus von Röntgenstrahlen ist,
- das Detektionselement (DT) dazu ausgelegt ist, Röntgenstrahlen zu detektieren, die den Schlitz (30) synchronisiert mit den Sendezyklen der Röntgenstrahlen durchqueren.

9. Detektionssystem (2) nach Anspruch 8, wobei der Träger (20) aus einem für Röntgenstrahlen szintillierenden Material besteht, wobei der Träger (20) imstande ist, Szintillationsphotonen zu senden, wenn der Träger der Röntgenstrahlenquelle ausgesetzt ist,

wobei das Detektionssystem (2) ferner eine Photodiode (PD) umfasst, die dazu ausgelegt ist, die Szintillationsphotonen zu detektieren und einen Photostrom zu senden, der aus den detektierten Szintillationsphotonen resultiert, so dass die Detektion und die Erfassung durch das Detektionselement (DT) von Röntgenstrahlen ausgelöst wird, die den Schlitz (30) durchqueren.

10. Detektionssystem (2) nach Anspruch 9, wobei die Verarbeitungseinheit (50) dazu ausgelegt ist, den von der Photodiode (PD) gesendeten Photostrom zu verwenden, um die Winkeldrehgeschwindigkeit des Trägers (V) während der Dauer der Impulse der gepulsten Quelle automatisch zu steuern.

11. Röntgenstrahlenabbildungssystem (3), umfassend:

ein Detektionssystem (2) nach einem der Ansprüche 4 bis 10;
eine Vielzahl von Erfassungselementen, die eine Erfassungsmatrix (M) bilden, die in einem Erfassungsfeld des Abbildungssystems platziert ist, wobei jedes Element der Erfassungsmatrix (M) dazu ausgelegt ist, eine Röntgenstrahlenstromakkumulation zu erfassen, wenn sich der Träger (20) des Kollimators des Detektionssystems um seine Achse (Δ) dreht, wobei die Erfassungsmatrix (M) dazu ausgelegt ist, ein radiografisches Bild ausgehend von den jeweiligen Erfassungen zu erfassen, die von den Erfassungselementen, die die Erfassungsmatrix (M) bilden, während eines gegebenen Zeitraums durchgeführt wurden.

12. Röntgenstrahlenabbildungssystem (3) nach Anspruch 11, das ferner umfasst:
eine ebene Blende (21), die zwischen der Röntgenstrahlenquelle (10) und dem Träger (20) platziert ist, die ein Sichtfeld begrenzt, dessen Projektion auf die Erfassungsmatrix (M) die Größe der Erfassungsmatrix (M) nicht überschreitet.

**13.** Röntgenstrahlenabbildungssystem (3) nach einem der Ansprüche 11 bis 12, das ein Detektionssystem (2) nach Anspruch 8 umsetzt, wobei:
die Erfassungsmatrix (M) dazu ausgelegt ist, radiografische Bilder synchronisiert mit den Sendezyklen der Röntgenstrahlen zu erfassen.

**14.** Röntgenstrahlenabbildungssystem (3) nach Anspruch 13, das ein Detektionssystem (2) nach Anspruch 9 umsetzt, wobei die Photodiode (PD) dazu ausgelegt ist, die Szintillationsphotonen zu detektieren und einen Photostrom zu senden, der aus den detektierten Szintillationsphotonen resultiert, so dass die Erfassung von mindestens einem der radiografischen Bilder durch die Erfassungsmatrix (M) ausgelöst wird.

**15.** Röntgenstrahlenabbildungssystem (3) nach Anspruch 14, das ein Detektionssystem (2) nach Anspruch 10 umsetzt, wobei die Verarbeitungseinheit (50) dazu ausgelegt ist, den von der Photodiode (PD) gesendeten Photostrom zu verwenden, um die Winkeldrehgeschwindigkeit des Trägers (V) während der Dauer der Impulse der gepulsten Quelle automatisch zu regeln.

**16.** Verfahren zur Umsetzung mittels eines Systems nach einem der Ansprüche 3 bis 15, wobei das Verfahren einen Detektionsschritt durch mindestens ein Detektionselement (DT) von Röntgenstrahlen umfasst, die den Schlitz (30) des Trägers der Kollimationsvorrichtung durchqueren, wenn der Träger um seine Achse (Δ) dreht und einem von der Quelle ausgehenden Röntgenstrahlenbündel ausgesetzt ist.

**17.** Verfahren nach Anspruch 16, wobei das System ein Röntgenstrahlen-Detektionssystem nach einem der Ansprüche 4 bis 10 ist, wobei der Träger mit einer festen Winkeldrehgeschwindigkeit dreht;

wobei der Detektionsschritt eine Detektion durch ein Detektionselement (DT) von Röntgenstrahlen umfasst, die den Schlitz (30) des Trägers zu zwei aufeinanderfolgenden Momenten durchqueren, einem ersten Moment (t_alpha), der einer ersten Winkelposition (alpha) des Schlitzes entspricht, und einem zweiten Moment (t_beta), der einer zweiten Winkelposition (alpha) des Schlitzes entspricht;
wobei das Verfahren ferner eine Bestimmung der projizierten Position (C) in der Hauptebene (P) des Trägers (20) des Detektionselements (DT) auf der Basis der ersten und zweiten Winkelposition durch die Verarbeitungseinheit (50) umfasst, wobei die erste und zweite Winkelposition auf der Basis des ersten und zweiten Moments und der Winkelgeschwindigkeit des Trägers bestimmt werden.

**18.** Verfahren nach Anspruch 16, wobei das System ein Röntgenstrahlen-Detektionssystem nach Anspruch 6 ist, wobei der Träger mit einer festen Winkeldrehgeschwindigkeit dreht, wobei das Verfahren umfasst:

- eine Detektion erzeugter elektrischer Impulse, wenn, beim Durchgang von einem beliebigen der Fenster zwischen dem Sender und dem Empfänger der optischen Gabel, der Empfänger die Lichtwelle empfängt;

wobei der Detektionsschritt eine Detektion von Röntgenstrahlen, die den Schlitz (30) des Trägers (20) durchqueren, für eine erste Winkelposition (alpha) des zweiten Trägers (20b) und eine zweite Winkelposition (beta) des zweiten Trägers (20b) durch ein Detektionselement (DT) umfasst,
wobei das Verfahren ferner eine Bestimmung der projizierten Position (C) in der Trägerhauptebene auf der Basis der ersten und zweiten Winkelposition durch die Verarbeitungseinheit (50) umfasst,
wobei die erste und zweite Winkelposition auf der Basis der Indexe der zwei Fenster erhalten werden, die die elektrischen Impulse erzeugt haben, die zeitlich den beiden aufeinanderfolgenden Momenten am nächsten sind, für die die Detektion durch das Detektionselement (DT) stattgefunden hatte.

**19.** Verfahren nach Anspruch 18, wobei das System ein Detektionssystem (2) nach Anspruch 10 ist, wobei die Verarbeitungseinheit (50) den von der Photodiode (PD) gesendeten Photostrom verwendet, um die Winkeldrehgeschwindigkeit des Trägers (V) während der Dauer der Impulse der gepulsten Quelle automatisch zu regeln.

**20.** Verfahren nach einem der Ansprüche 17 bis 19, umfassend einen Schritt des Bestimmens, durch die Verarbeitungseinheit (50), des Abstands (D) zwischen einer Ebene, die das Detektionselement (DT) enthält und parallel zur Hauptebene (P) des Trägers (20) ist, und der Röntgenstrahlenquelle (10) ausgehend von einer Bestrahlungsdauer (tirr) des Detektionselements (DT), der Winkeldrehgeschwindigkeit des Trägers (V), des Abstands (D) zwischen der Röntgenstrahlenquelle (10) und dem Träger (20), der Breite des Schlitzes (1) und dem Abstand r zwischen dem Schnitt der Drehachse (Δ) des Trägers (20) und der projizierten Position (C) in der Hauptebene (P) des Trägers (20) .

**21.** Verfahren nach Anspruch 16, wobei das System ein Röntgenstrahlenabbildungssystem (3) nach einem der Ansprüche 11 bis 12 ist, wobei der Träger mit einer festen Winkeldrehgeschwindigkeit dreht, wobei das Verfahren die folgenden Schritte umfasst:

- eine Röntgenstrahlendetektion durch die Erfassungselemente der Erfassungsmatrix während der Drehung des Trägers (20);
- eine Erfassung eines radiografischen Bilds ausgehend von den jeweiligen Detektionen, die von den Erfassungselementen der Detektionsmatrix (M) während eines gegebenen Zeitraums durchgeführt wurden.

**22.** Verfahren nach Anspruch 16, wobei das System ein Röntgenstrahlenabbildungssystem (3) nach Anspruch 14 ist, wobei der Träger mit einer festen Winkeldrehgeschwindigkeit dreht, wobei das Verfahren die folgenden Schritte umfasst:

- Detektieren von Röntgenstrahlen durch die Erfassungselemente der Erfassungsmatrix während der Drehung des Trägers (20);
- Senden von Szintillationsphotonen durch den Träger (20);
- Detektieren der Szintillationsphotonen durch die Photodiode (PD),
- Senden, durch die Photodiode, eines Photostroms, der aus den detektierten Szintillationsphotonen resultiert, so dass die Erfassung mindestens eines der radiografischen Bilder durch die Erfassungsmatrix (M) ausgelöst wird,
- Erfassen eines radiografischen Bildes ausgehend von den jeweiligen durchgeführten Detektionen durch die Erfassungselemente der Detektionsmatrix (M) während eines gegebenen Zeitraums.

**23.** Verfahren nach Anspruch 22, wobei das System ein Röntgenstrahlenabbildungssystem (3) nach Anspruch 15 ist, wobei die Verarbeitungseinheit (50) den von der Photodiode (PD) gesendeten Photostrom verwendet, um die Winkeldrehgeschwindigkeit des Trägers (V) während der Dauer der Impulse der gepulsten Quelle automatisch zu regeln, so dass die Erfassung eines radiografischen Bild ausgehend von den jeweiligen durchgeführten Detektionen durch die Erfassungselemente der Erfassungsmatrix (M) über die Röntgenstrahlen-Sendemodi der Sendezyklen der gepulsten Quelle zeitlich gefenstert wird.

**Claims**

**1.** A collimation device (1) for an X-ray detection system, the collimation device (1) comprising:
a collimator comprising a substantially planar support (20) made of a material with partial or zero radio transparency, the support (20) being rotatably movable about an axis (Δ) of rotation which passes through the support and which is perpendicular to a first face of the support (20) which acts as an X-ray plane of incidence referred to as the main plane of the support (P), said support (D) being provided on the first face with a single slit (30) which is completely transparent to X-rays and which is configured to generate an X-ray flow when the collimator is exposed to an X-ray source (10), said slit (30) extending longitudinally in the main plane of the support along an axis located at a non-zero distance (d) from the axis of rotation (Δ), said slit (30) extending through the entire thickness of the support (20).

**2.** The collimation device (1) as claimed in claim 1, wherein the slit (30) has a projection of rectangular or trapezoidal shape in the main plane of the support (P).

**3.** An X-ray detection system (2), the detection system (2) comprising:

- an X-ray source (10) configured to emit an X-ray beam;
- a collimation device (1) as claimed in any one of the preceding claims, placed so as to receive the X-ray beam on the plane of incidence of the support;
- a drive device configured to rotate the support (20) of the collimation device about its axis (Δ) with a fixed angular speed of rotation (V);
- at least one X-ray detection element placed in an acquisition field opposite the support (20) with respect to the X-ray source (10) so as to acquire X-rays when a portion of the X-ray beam, passing through the slit (30) in the support (20) from the first face of the support to the opposite face, reaches the considered detection element during the rotation of the support.

**4.** The detection system (2) as claimed in claim 3,

wherein one detection element (DT) from among said at least one X-ray detection element is configured to detect X-rays passing through the slit (30) at two successive times while the support (20) is rotating, a first time (t_alpha) corresponding to a first angular position (alpha) of the slit and a second time (t_beta) corresponding to a second angular position (beta) of the slit (30), the first and second angular positions defining in the main plane of the support (P) a point of intersection (C) corresponding to the projected position (PT) in the main plane of the support (20) of the detection element (DT);

the detection system (2) comprising a processing unit (50) configured to determine the projected position (C) in the main plane of the support (P) of the detection element (DT) from the first and second angular positions.

5. The detection system as claimed in claim 4, the processing unit being configured to determine the first and second angular positions based on the first and second times and the angular speed of the support.

6. The detection system (2) as claimed in claim 4, further comprising:

- a second support (20b) integral with the support (20) or forming an integral part of the support (20), comprising a series of N windows uniformly distributed angularly around a circular periphery centered with respect to the axis (Δ) of rotation of the support, said series of N windows comprising a reference window of different size from the other windows, the other windows being indexed with respect to this reference window,
- an optical fork, the optical fork being provided with an emitter configured to emit a light wave and a detector configured to detect the light wave, said fork being configured to generate electrical pulses when, upon any one of the windows passing between the emitter and the receiver, the receiver receives the light wave, the processing unit (50) being configured to determine the first and second angular positions based on the indexes of the two windows (20b) that have produced the electrical pulses which are temporally closest to the two successive times for which the detection by the detection element (DT) has taken place.

7. The detection system (2) as claimed in any one of claims 4 to 6, wherein the processing unit (50) is configured to:

- determine the distance between a plane passing through the detection element (DT) and parallel to the main plane of the support (P) and the X-ray source (10), in the case where the slit is rectangular in shape, from a duration of irradiation of the detection element (DT), from the angular speed of rotation of the support (V), from the distance (D) between the X-ray source (10) and the support (20) and from the width (1) of the slit and from the distance r between the intersection of the axis of rotation (Δ) of the support (20) and the projected position (C) in the main plane (P) of the support (20).

8. The detection system (2) as claimed in any one of claims 4 to 7, wherein:

- the X-ray source (10) is a pulsed source configured to emit X-rays in emission cycles composed of two modes used alternately, one being an X-ray emission mode, the other being an X-ray non-emission mode,
- the detection element (DT) is configured to detect X-rays passing through the slit (30) in synchronization with the X-ray emission cycles.

9. The detection system (2) as claimed in claim 8, wherein the support (20) is made of an X-ray scintillating material, said support (20) being able to emit scintillation photons when the support is exposed to the X-ray source, said detection system (2) further comprising a photodiode (PD) configured to detect the scintillation photons and emit a photocurrent resulting from the detected scintillation photons so as to trigger the detection and acquisition by the detection element (DT) of X-rays passing through the slit (30).

10. The detection system (2) as claimed in claim 9, wherein the processing unit (50) is configured to use the photocurrent emitted by the photodiode (PD) to automatically feedback-control the angular speed of rotation of the support (V) with respect to the duration of the pulses from the pulsed source.

11. An X-ray imaging system (3) comprising:

a detection system (2) as claimed in any one of claims 4 to 10;
a plurality of acquisition elements forming an acquisition matrix (M) placed in an acquisition field of the imaging system, each element of the acquisition matrix (M) being configured to acquire an X-ray flow build-up when the support (20) of the collimator of the detection system is rotating about its axis (Δ), the acquisition matrix (M) being configured to acquire a radiographic image on the basis of the acquisitions respectively made by the acquisition elements constituting the acquisition matrix (M) during a given period of time.

12. The X-ray imaging system (3) as claimed in claim 11, further comprising:
a planar diaphragm (21) placed between the X-ray source (10) and the support (20), delimiting a field of view whose projection on the acquisition matrix (M) does not exceed the size of the acquisition matrix (M).

13. The X-ray imaging system (3) as claimed in any of claims 11 and 12 implementing a detection system (2) as claimed in claim 8, wherein:
the acquisition matrix (M) is configured to acquire radiographic images in synchronization with the X-ray emission cycles.

14. The X-ray imaging system (3) as claimed in claim 13, implementing a detection system (2) as claimed in claim 9, wherein the photodiode (PD) is configured to detect the scintillation photons and emit a photocurrent resulting from the detected scintillation photons so as to trigger the acquisition by the acquisition matrix (M) of at least one of the radiographic images.

15. The X-ray imaging system (3) as claimed in claim 14, implementing a detection system (2) as claimed in claim 10, wherein the processing unit (50) is configured to use the photocurrent emitted by the photodiode (PD) to automatically feedback-control the angular speed of rotation of the support (V) with respect to the duration of the pulses from the pulsed source.

16. A method implemented by means of a system as claimed in any of claims 3-15, the method comprising a step for detecting, by at least one detection element (DT), X-rays passing through the slit (30) of the support of the collimation device when the support is rotating around its axis (Δ) and exposed to an X-ray beam emitted from the source.

17. The method as claimed in claim 16, wherein the system is an X-ray detection system as claimed in any of claims 4-10, the support being rotated at a fixed angular speed of rotation;

the detection step comprising a detection by a detection element (DT) of X-rays passing through the slit (30) of the support at two successive times, a first time (t-alpha) corresponding to a first angular position (alpha) of the slit and a second time (t_beta) corresponding to a second angular position (alpha) of the slit;
the method further comprising a determination by the processing unit (50) of the projected position (C) in the main plane (P) of the support (20) of the detection element (DT) based on the first and second angular positions, the first and second angular positions being determined based on the first and second times and the angular speed of the support.

18. The method as claimed in claim 16, wherein the system is an X-ray detection system as claimed in claim 6, the support being rotated at a fixed angular speed of rotation, the method comprising:

- detecting electrical pulses produced when, upon any one of the windows passing between the emitter and the receiver of the optical fork, the receiver receives the light wave;

the detection step comprising a detection by a detection element (DT) of X-rays passing through the slit (30) of the support (20) for a first angular position (alpha) of the second support (20b) and a second angular position (beta) of the second support (20b),
the method further comprising a determination by the processing unit (50) of the projected position (C) in the main plane of the support based on the first and second angular positions,
the first and second angular positions being obtained based on the indexes of the two windows that have produced the electrical pulses which are temporally closest to the two successive times for which the detection by the detection element (DT) has taken place.

19. The method as claimed in claim 18, the system being a detection system (2) as claimed in claim 10, wherein the processing unit (50) uses the photocurrent emitted by the photodiode (PD) to automatically feedback-control the angular speed of rotation of the support (V) with respect to the duration of the pulses from the pulsed source.

20. The method as claimed in any one of claims 17-19, comprising a step for determining, by the processing unit (50), the distance (D) between a plane containing the detection element (DT) and parallel to the main plane (P) of the support (20) and the X-ray source (10) from a duration of irradiation (tirr) of the detection element (DT), from the angular speed of rotation of the support (V), from the distance (D) between the X-ray source (10) and the support (20), from the width of the slit (1) and from the distance r between the intersection of the axis of rotation (∆) of the support (20) and the projected position (C) in the main plane (P) of the support (20) .

21. The method as claimed in claim 16, the system being an X-ray imaging system (3) as claimed in any of claims 11 and 12, the support being rotated at a fixed angular speed of rotation, the method comprising the following steps:

- detecting X-rays by the acquisition elements of the acquisition matrix during the rotation of the support (20);
- acquiring a radiographic image on the basis of the detections respectively made by the acquisition elements of the detection matrix (M) during a given period of time.

22. The method as claimed in claim 16, the system being an X-ray imaging system (3) as claimed in claim 14, the support being rotated at a fixed angular speed of rotation, the method comprising the following steps:

- detecting X-rays by the acquisition elements of the acquisition matrix during the rotation of the support (20);
- emitting scintillation photons by the support (20);
- detecting scintillation photons by the photodiode (PD);
- emitting by the photodiode a photocurrent resulting from the detected scintillation photons so as to trigger the acquisition by the acquisition matrix (M) of at least one of the radiographic images;
- acquiring a radiographic image on the basis of the detections respectively made by the acquisition elements of the detection matrix (M) during a given period of time.

23. The method as claimed in claim 22, the system being an X-ray imaging system (3) as claimed in claim 15, wherein the processing unit (50) uses the photocurrent emitted by the photodiode (PD) to automatically feedback-control the angular speed of rotation of the support (V) with respect to the duration of the pulses of the pulsed source such that the acquisition of a radiographic image on the basis of the detections respectively made by the acquisition elements of the acquisition matrix (M) is temporally windowed over the X-ray emission modes of the emission cycles of the pulsed source.

**FIG. 1**

| Material | K-edge keV | 40keV mm | 60keV mm | 80keV mm | 100keV mm |
|---|---|---|---|---|---|
| NaI | 33.2 | 0.43 | 1.3 | 2.7 | 4.9 |
| CsI | 33.2 | 0.29 | 0.8 | 1.8 | 3.3 |
| CdWO₄ | 69.5 | 0.33 | 0.9 | 0.5 | 0.9 |
| BGO | 90.5 | 0.38 | 1.1 | 2.2 | 1.1 |
| LSO | 63.3 | 0.55 | 1.6 | 0.7 | 1.3 |
| PreLude (LYSO) | 63.3 | 0.58 | 1.65 | 0.8 | 1.4 |

**FIG. 2**

**FIG. 3**

**FIG. 4**

**FIG. 5**

**FIG. 6A**

0°    360°/13         alpha~ (11/ 39) *360°        beta~ (27/ 39) *360°        360°    θ

**FIG. 6B**

FIG. 7

| Position | Δt | π–(beta-alpha) | r |
|----------|------|---------|---------|
| P1 | 26.1ms | 18.7° | 12.9mm |
| P2 | 23.7ms | 32.5° | 7.2mm |
| P3 | 20.3ms | 54.1° | 4.4mm |

| Position | Δt | π–(beta-alpha) | r |
|----------|------|---------|---------|
| P1 | 17.1ms | 19.5° | 11.8mm |
| P2 | 15.7ms | 32.2° | 7.2mm |
| P3 | 13.3ms | 54.5° | 4.4mm |

**FIG. 8**

**FIG. 9**

FIG. 10

**FIG. 11**

FIG. 12

FIG. 13

**FIG. 14A**

**FIG. 14B**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2841118
- US 6490475 B
- US 7065393 B
- FR 3042881
- WO 2017072125 A1
- FR 3028039 A1
- WO 2016071645 A1